# EUROPEAN PATENT APPLICATION

(11) **EP 4 813 289 A1**
(43) Date of publication of application: **30.09.2026**
(21) Application number: 24893472.1
(22) Date of filing: 20.11.2024
(51) Int. Cl.: A61B 5/024, A61B 5/0245, A61B 5/318, A61B 5/346, A61B 5/369, A61B 5/372, A61B 5/0533, A61B 5/00, G06F 18/00, G06F 18/24, G06F 18/25

(54) **MULTI-MODAL HUMAN PHYSIOLOGICAL DATA CLASSIFICATION MODEL AND TRAINING METHOD THEREFOR, MULTI-MODAL HUMAN PHYSIOLOGICAL DATA CLASSIFICATION METHOD, AND DEVICE**

(30) Priority: 23.11.2023 CN 202311576945; 30.11.2023 CN 202311635615; 22.12.2023 CN 202311790144; 26.12.2023 CN 202311810607
(71) Applicant: Kingfar International Inc., Beijing 100085 (CN); Nanjing Kingfar Health Technology Inc., Nanjing, Jiangsu 211112 (CN)
(72) Inventor: ZHAO, Qichao, Beijing 100085 (CN); WANG, Qingju, Beijing 100085 (CN); YANG, Ran, Beijing 100085 (CN)
(74) Representative: M. Zardi & Co S.A.
(86) International application number: PCT/CN2024/133264
(87) International publication number: WO 2025/108316

(57) **Abstract**

The present disclosure relates to the field of artificial intelligence technologies, and in particular, to a multimodal human physiological data classification model and a training method therefor, a multimodal human physiological data classification method and a device, aiming to solve a classification problem in scenarios involving miss multimodal physiological data. The classification model includes: a multi-headed self-attention module, a normalization module, a fusion expert system, and a decision module. The fusion expert system includes four expert subsystems: an electroencephalogram expert subsystem, an electrocardiogram expert subsystem, an electrodermal activity expert subsystem, and a multimodal synchronous fusion expert subsystem. The multi-headed self-attention module is configured to perform feature extraction on multimodal synchronous data. The normalization module is configured to generate normalized feature data based on the extracted feature data. The four expert subsystems are configured to perform classification tasks based on the normalized feature data corresponding to electroencephalogram data, electrocardiogram data, electrodermal activity data and multimodal synchronous fusion data, respectively. The decision module is configured to calculate a final classification result based on the above four classification results. The present disclosure can improve the accuracy of classification results in scenarios involving miss multimodal data.

## Description

### CROSS-REFERENCE TO RELATED APPLICATIONS

This application claims priorities to Chinese Patent Applications No. 202311635615.7, filed on November 30, 2023 and entitled "CLASSIFICATION MODEL FOR MULTIMODAL HUMAN PHYSIOLOGICAL DATA, TRAINING METHOD FOR CLASSIFICATION MODEL FOR MULTIMODAL HUMAN PHYSIOLOGICAL DATA, CLASSIFICATION METHOD FOR MULTIMODAL HUMAN PHYSIOLOGICAL DATA, AND DEVICE", No. 202311576945.3, filed on November 23, 2023 and entitled "TRAINING METHOD FOR PHYSIOLOGICAL STATE PREDICTION MODEL, AND SAMPLE DATA GENERATION AND PREDICTION METHOD", No. 202311790144.7, filed on December 22, 2023 and entitled "HUMAN-FACTOR INTELLIGENCE-BASED PHYSIOLOGICAL SIGNAL PROCESSING METHOD AND APPARATUS, AND ELECTRONIC DEVICE", and No. 202311810607.1, filed on December 22, 2023 and entitled "HUMAN-FACTOR INTELLIGENCE-BASED PHYSIOLOGICAL SIGNAL PROCESSING METHOD AND APPARATUS, AND ELECTRONIC DEVICE", the entire contents of which are incorporated herein by reference.

### FIELD

The present disclosure relates to the fields of human factors engineering and artificial intelligence technologies, and in particular, to a classification model for multimodal human physiological data, a training method for a classification model for multimodal human physiological data, a classification method for multimodal human physiological data, and a device.

### BACKGROUND

In human factors-related research and application scenarios, it is often desirable use a neural network model to classify a current physiological or psychological state of a user based on human physiological data (such as electroencephalogram and electrocardiogram, etc.), for example, to determine whether the user is currently in a state of pleasure or anger.

However, during physiological data collection, poor sensor or electrode contact, user movement, interference from other devices, and other factors may lead to missing data in collected physiological data, which in turn causes a deviation in the model's classification results.

### SUMMARY

The present disclosure provides a classification model for multimodal human physiological data, a training method for a classification model for multimodal human physiological data, a classification method for multimodal human physiological data, and a device.

In a first aspect of the present disclosure, a classification model for multimodal human physiological data is provided. The classification model includes: a multi-headed self-attention module, a normalization module, a fusion expert system, and a decision module. The fusion expert system includes an electroencephalogram expert subsystem, an electrocardiogram expert subsystem, an electrodermal activity expert subsystem, and a multimodal synchronous fusion expert subsystem; the multi-headed self-attention module is configured to perform feature extraction on inputted multimodal synchronous data and output the extracted feature data to the normalization module, the multimodal synchronous data including: a physiological indicator of a user, and the physiological indicator including electroencephalogram (EEG) data, electrocardiogram (ECG) data, and electrodermal activity (EDA) data; the normalization module is configured to generate normalized feature data based on the extracted feature data and output the normalized feature data to the fusion expert system; the electroencephalogram expert subsystem is configured to perform a classification task based on the normalized feature data corresponding to the electroencephalogram data of the user to obtain a first classification result; the electrocardiogram expert subsystem is configured to perform a classification task based on the normalized feature data corresponding to the electrocardiogram data of the user to obtain a second classification result; the electrodermal activity expert subsystem is configured to perform a classification task based on the normalized feature data corresponding to the electrodermal activity data of the user to obtain a third classification result; the multimodal synchronous fusion expert subsystem is configured to perform a classification task based on the normalized feature data corresponding to the multimodal synchronous data to obtain a fourth classification result; and the decision module is configured to calculate a final classification result based on the first classification result, the second classification result, the third classification result, the fourth classification result, and a weight corresponding to each of the first classification result, the second classification result, the third classification result, and the fourth classification result.

In a second aspect of the present disclosure, provided is a training method for a classification model for multimodal human physiological data, applicable to the classification model for multimodal human physiological data as described above. The training method includes: training the electroencephalogram expert subsystem and the multi-headed self-attention module using an electroencephalogram training set, and freezing a weight of the electrocardiogram expert subsystem, a weight of the electrodermal activity expert subsystem, and a weight of the multimodal synchronous fusion expert subsystem; training the electrocardiogram expert subsystem using an electrocardiogram training set, and freezing a weight of the multi-headed self-attention module, a weight of the electroencephalogram expert subsystem, the weight of the electrodermal activity expert subsystem, and the weight of the multimodal synchronous fusion expert subsystem; training the electrodermal activity expert subsystem using an electrodermal activity training set, and freezing the weight of the multi-headed self-attention module, the weight of the electroencephalogram expert subsystem, the weight of the electrocardiogram expert subsystem, and the weight of the multimodal synchronous fusion expert subsystem; and training the classification model using a multimodal random masking training set, to adjust the weight of the multi-headed self-attention module, the weight of the electroencephalogram expert subsystem, the weight of the electrocardiogram expert subsystem, the weight of the electrodermal activity expert subsystem, and the weight of the multimodal synchronous fusion expert subsystem.

In a third aspect of the present disclosure, a classification method for multimodal human physiological data is provided. The classification method comprises performing a classification task using the classification model for multimodal human physiological data as described above.

In a fourth aspect of the present disclosure, a computer-readable storage device is provided. The computer-readable storage device stores a computer program. The computer program is capable of being loaded and executed by a processor to implement the method as described above.

The present disclosure has the following beneficial effects.

The present disclosure makes improvements on the original Vision Language Pretrained Model (VLMo) to better adapt the classification model to human physiological data. This enables the model to fully utilize information of each modality (the EEG data, the ECG data, and the EDA data) while achieving multimodal fusion of data. As a result, the model's performance not to rely solely on the quality of data from a single modality, thereby improving the robustness of the model.

In order to allow the classification model to maintain its good performance in scenarios with missing data, during a model training stage, the preprocessed data is artificially masked to simulate a data missing scenario. This allows the trained model to generalize to the masked part, i.e., the data-missing part in actual scenarios, thereby obtaining the most reliable result.

Considering that the EEG signal contain information in three dimensions, i.e., time-domain information, frequency-domain information, and spatial information, the present disclosure, after transforming the EEG signal into the frequency-domain information, obtains a multispectral-like image based on different leads according to positions of electrodes at which the electroencephalogram signal is collected. Then, it segments the multispectral-like image, and generates the electroencephalogram data as input data of the model based on segmented data. This method fully explores information contained in a multi-dimensional space and improves data utilization efficiency.

### BRIEF DESCRIPTION OF THE DRAWINGS

FIG. 1 is a schematic diagram showing main composition of a classification model for multimodal human physiological data according to an embodiment of the present disclosure;
FIG. 2 is a schematic diagram showing main steps of a training method for a classification model for multimodal human physiological data according to an embodiment of the present disclosure;
FIG. 3 is a schematic diagram of a system architecture provided by an implementation of the present disclosure;
FIG. 4 is a schematic flowchart of a method for generating electroencephalogram signal sample data provided by an implementation of the present disclosure;
FIG. 5 is a schematic flowchart of a training method for a physiological state prediction model provided by an implementation of the present disclosure;
FIG. 6 is a schematic diagram of a physiological state prediction model provided by an implementation of the present disclosure;
FIG. 7 is a schematic diagram of a structure of a recognition model provided by an embodiment of the present disclosure;
FIG. 8 is a schematic diagram of a structure of a channel attention network provided by an embodiment of the present disclosure;
FIG. 9 is a schematic diagram of a structure of a spatial attention network provided by an embodiment of the present disclosure;
FIG. 10 is a schematic diagram of a structure of another recognition model provided by an embodiment of the present disclosure;
FIG. 11 is a schematic diagram of a structure of a physiological signal recognition network provided by an embodiment of the present disclosure;
FIG. 12 is a schematic diagram of a time window provided according to an embodiment of the present disclosure;
FIG. 13 is a schematic diagram of a structure of a neural network model provided according to an embodiment of the present disclosure;
FIG. 14 is a schematic diagram of an original electrocardiogram signal provided according to an embodiment of the present disclosure;
FIG. 15 is a schematic diagram of a target electrocardiogram signal provided according to an embodiment of the present disclosure;
FIG. 16 is a schematic diagram of another target electrocardiogram signal provided according to an embodiment of the present disclosure; and
FIG. 17 is a schematic diagram of a structure of an electronic device provided according to an embodiment of the present disclosure.

### DETAILED DESCRIPTION

The preferred implementations of the present disclosure will be described below with reference to the accompanying drawings. It should be understood by those skilled in the art that these implementations are only used to explain the technical principles of the present disclosure, and are not intended to limit the scope of the present disclosure.

In order to make the purpose, technical solution and advantages of the embodiments of the present disclosure clearer, the following will provide a clear and complete description of the technical solution in the embodiments of the present disclosure in combination with the accompanying drawings in the embodiments of the present disclosure. Obviously, the described embodiments are some of the embodiments of the present disclosure, rather than all of them. Based on the embodiments of the present disclosure, all other embodiments obtained by those skilled in the art without any inventive effort fall within the scope of the present disclosure.

It should be noted that in the description of the present disclosure, the terms "first" and "second" are only used for descriptive purposes, and cannot be understood as indicating or implying relative importance of the described devices, elements, or parameters. Therefore, the features associated with "first" and "second" may not be understood as limiting the present disclosure. In addition, in the present disclosure, the term "and/or" only represents a relationship between correlated objects, including three relationships. For example, "A and/or B" may mean three situations: A only, B only, or both A and B. In addition, unless otherwise specified, the character "/" in the present disclosure generally represents an "or" relationship between the correlated objects preceding and succeeding the symbol.

### First embodiment

Microsoft has provided a unified vision language model, Vision Language Pretrained Model (VLMo). VLMo is equivalent to a mixture-of-experts model, and its Feed Forward Network (FFN) part has three modes: a vision mode for image encoding (V-FFN), a language mode for text encoding (L-FFN), and a vision language mode for image-text fusion (VL-FFN).

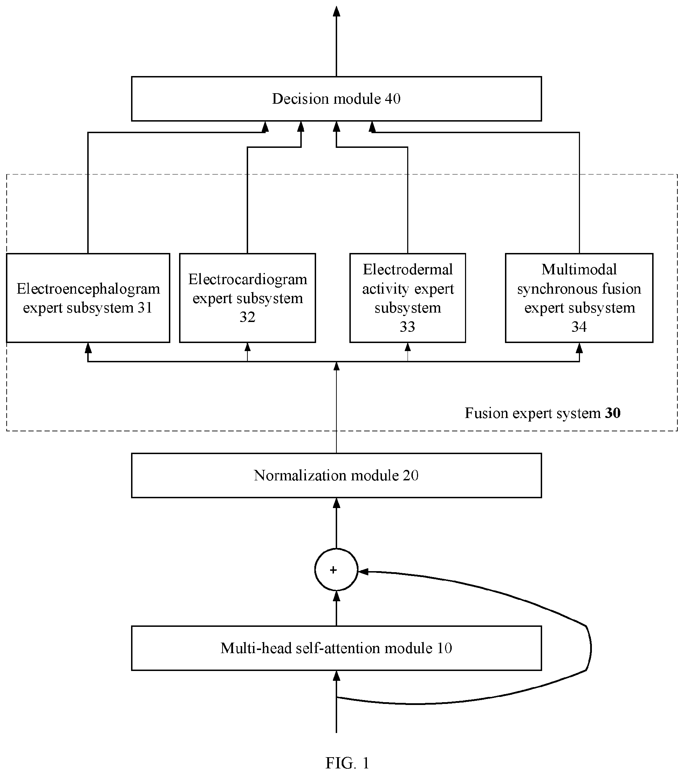

FIG. 1 is a schematic diagram showing main composition of a classification model for multimodal human physiological data according to an embodiment of the present disclosure. As shown in FIG. 1, the classification model of this embodiment includes a multi-headed self-attention module 10, a normalization module 20, a fusion expert system 30, and a decision module 40.

The fusion expert system 30 includes: an electroencephalogram expert subsystem 31, an electrocardiogram expert subsystem 32, an electrodermal activity expert subsystem 33, and a multimodal synchronous fusion expert subsystem 34. The multimodal synchronous data includes a physiological indicator of a user. The physiological indicator includes electroencephalogram data, electrocardiogram data, and electrodermal activity data, and all the data are collected within one time period.

In this embodiment, the multi-headed self-attention module 10 is configured to perform feature extraction on inputted multimodal synchronous data and output the extracted feature data to the normalization module 20.

In this embodiment, the normalization module 20 is configured to normalize the extracted feature data and output the normalized feature data to the fusion expert system 30.

In this embodiment, the electroencephalogram expert subsystem 31 is configured to perform a classification task based on the normalized feature data corresponding to the electroencephalogram data of the user to obtain a first classification result. The electrocardiogram expert subsystem 32 is configured to perform a classification task based on the normalized feature data corresponding to the electrocardiogram data of the user to obtain a second classification result. The electrodermal activity expert subsystem 33 is configured to perform a classification task based on the normalized feature data corresponding to the electrodermal activity data of the user to obtain a third classification result. The multimodal synchronous fusion expert subsystem 34 is configured to perform a classification task based on the normalized feature data corresponding to the multimodal synchronous data to obtain a fourth classification result.

In this embodiment, the decision module 40 is configured to calculate a final classification result based on the first classification result, the second classification result, the third classification result, the fourth classification result, and a weight corresponding to each of the classification results.

Specifically, the final classification result is calculated according to the following equation (1): $G = {W}_{1} * {f}_{1} + {W}_{2} * {f}_{2} + {W}_{3} * {f}_{3} + {W}_{4} * {f}_{4}$ where G denotes the final classification result; f₁, f₂, f₃ and f₄ respectively denote the first classification result, the second classification result, the third classification result, and the fourth classification result; and W₁ , W₂ , W₃, and W₄ each denote a weight.

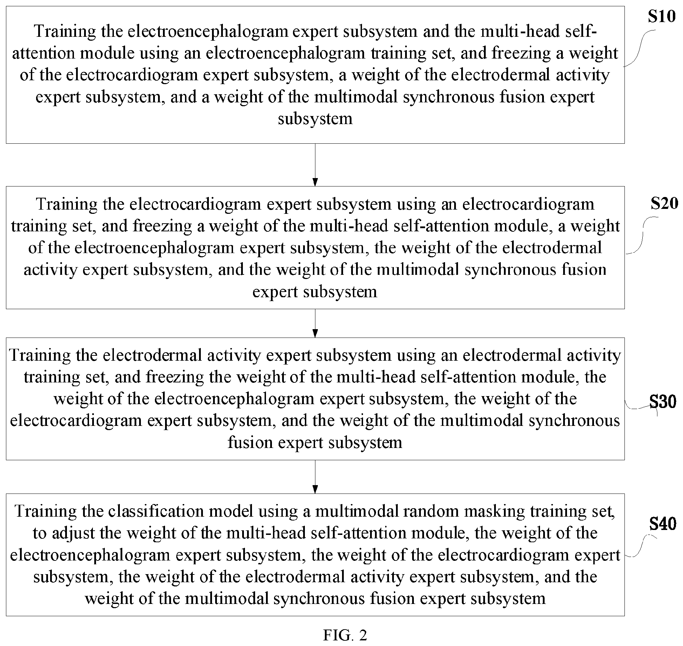

FIG. 2 is a schematic diagram showing main steps of a training method for a classification model for multimodal human physiological data according to an embodiment of the present disclosure. The training method of this embodiment is applicable to the classification model for multimodal human physiological data as described above. As shown in FIG. 2, the training method of this embodiment includes steps S10 to S40.

At step S10, the electroencephalogram expert subsystem and the multi-headed self-attention module are trained using an electroencephalogram training set, and a weight of the electrocardiogram expert subsystem, a weight of the electrodermal activity expert subsystem, and a weight of the multimodal synchronous fusion expert subsystem are frozen.

At step S20, the electrocardiogram expert subsystem is trained using an electrocardiogram training set, and a weight of the multi-headed self-attention module, a weight of the electroencephalogram expert subsystem, the weight of the electrodermal activity expert subsystem, and the weight of the multimodal synchronous fusion expert subsystem are frozen.

At step S30, the electrodermal activity expert subsystem is trained using an electrodermal activity training set, and the weight of the multi-headed self-attention module, the weight of the electroencephalogram expert subsystem, the weight of the electrocardiogram expert subsystem, and the weight of the multimodal synchronous fusion expert subsystem are frozen.

At step S40, the classification model is trained using a multimodal random masking training set, to adjust the weight of the multi-headed self-attention module, the weight of the electroencephalogram expert subsystem, the weight of the electrocardiogram expert subsystem, the weight of the electrodermal activity expert subsystem, and the weight of the multimodal synchronous fusion expert subsystem.

In an optional embodiment, the training method for the classification model further includes steps S1 to S4 for data preprocessing.

At step S1, electroencephalogram data containing time-domain information, frequency-domain information, and spatial information is generated according to an electroencephalogram signal of a user and positions of electrodes corresponding to the electroencephalogram signal, thereby obtaining the electroencephalogram training set.

At step S2, denoising processing is performed on an electrocardiogram signal of the user, and the denoised electrocardiogram signal is aligned with the electroencephalogram signal of the user according to temporal information to generate the electrocardiogram training set.

For a specific user, only the electroencephalogram signal, the electrocardiogram signal, and the electrodermal activity signal that are collected within the same time period are meaningfully correlated. Therefore, these data need to be temporally aligned.

At step S3, denoising processing is performed on an electrodermal activity signal of the user, and the denoised electrodermal activity signal is aligned with the electroencephalogram signal of the user according to the temporal information to generate the electrodermal activity training set.

At step S4, random masking processing is performed on the electroencephalogram training set, the electrocardiogram training set, and the electrodermal activity training set to simulate a data missing scenario, to generate the multimodal random masking training set.

Preferably, the above step S1 may specifically include steps S11 to S14.

At step S11, fast Fourier transform is performed on the electroencephalogram signal of the user to extract the frequency-domain information.

At step S12, data within a frequency band of interest is selected from the frequency-domain information.

For example, for a cognitive load task, data within theta (4Hz to 7Hz), alpha (8Hz to 13Hz), and beta (13Hz to 30Hz) frequency bands, which are closely related to memory, can be used.

At step S13, a multispectral-like image based on different leads is obtained according to the data within the frequency band of interest and positions of electrodes at which the electroencephalogram signal is collected.

At step S14, the multispectral-like image is segmented, and the electroencephalogram data of the user is generated according to segmented data.

The electroencephalogram signal collected by an electroencephalograph is time-series data. A conventional operation is to transform the signal into a frequency domain to use the time-domain information and the frequency-domain information. However, this method ignores the spatial information contained in the electroencephalogram signal. In order to make full use of information of various dimensions, the present disclosure performs special processing on the electroencephalogram signal to obtain information in three dimensions, i.e., the time-domain information, the frequency-domain information, and the spatial information, thereby improving an information utilization.

In a preferred embodiment, the above step S11 may further include the following sub-steps.
(1) The positions of the electrodes at which the electroencephalogram signal is collected are projected from a three-dimensional space to a two-dimensional surface to obtain two-dimensional position information of each electrode. Specifically, an azimuthal equidistant projection in the Cartesian coordinate system is performed to transform the positions of the electrodes at which the electroencephalogram signal is collected from the three-dimensional space to the two-dimensional surface, such that the transformed data of respective leads retain a spatial topological relationship.
   The electroencephalogram (EEG) signal is time-series data collected by an EEG cap worn on the head. Electrodes of the EEG cap have a spatial topology in their distribution on the scalp. Therefore, the collected electroencephalogram signal contains both temporal information and spatial information. In order to transform a spatial distribution activity map into a two-dimensional image, it is necessary to project the positions of the electrodes from the three-dimensional space to the two-dimensional surface. For this purpose, the azimuthal equidistant projection in the Cartesian coordinate system is performed for the transformation. Compared with other projection methods, this projection method can not only transform three-dimensional coordinate points into two-dimensional points, but can also retain a distance relationship between the three-dimensional points, such that the transformed EEG data of respective leads retain a spatial topological relationship.
(2) Distances between each electrode and other surrounding electrodes is calculated according to the two-dimensional position information.
(3) For data of each lead in the data within the frequency band of interest, corresponding weights are set for data of other leads according to the distances, thereby obtaining the multispectral-like image based on the different leads.

In one implementation, the above step S14 may further include the following sub-steps.
(1) The multispectral-like image is segmented to obtain *N = H * W* / *P²* patches of a same size. H, W, C, and P denote a height, width, number of channels of the image, and a size of each of the *N = H * W* / *P²* patches, respectively.
(2) For each patch, segmented data corresponding to the patch is obtained by flattening the patch into a vector, obtaining Patch embedding through a linear projection, and adding an [I_CLS] token is position encoding for the patch. The position encoding is used to record a position of the patch in the image during the segmenting.
(3) The electroencephalogram data of the user is generated according to the segmented data.

In the present disclosure, the multispectral-like image is treated as a picture for processing. The width and the height of the picture represent spatial distribution of cerebral cortex activity. In this embodiment, the EEG data is processed into a 32×32 grid structure (P=32).

Although the steps in the above embodiment are described in the above sequence, those skilled in the art can understand that in order to achieve the effect of this embodiment, the different steps do not need to be executed in this sequence, and they can be executed simultaneously (in parallel) or in reverse order. These simple changes all fall within the scope of the present disclosure.

Further, based on the above classification model, the present disclosure further provides an embodiment of a classification method for multimodal human physiological data. In this embodiment, the classification task is performed using the classification model for multimodal human physiological data as described above.

Before the classification task is performed, it is also necessary to preprocess the electroencephalogram signal of the user, the electrocardiogram signal of the user, and the electrodermal activity signal of the user, respectively, referring to the preprocessing steps S1 to S3 in the above embodiments of the training method. That is, the electroencephalogram data containing the time-domain information, the frequency-domain information, and the spatial information is generated according to the electroencephalogram signal of the user and the positions of the electrodes corresponding to the electroencephalogram signal, and denoising processing and alignment processing are performed on the electrocardiogram signal and electrodermal activity signal.

Further, the present disclosure further provides an embodiment of a computer-readable storage device. The storage device of this embodiment stores a computer program that is capable of being loaded and executed by a processor to implement the method as described above.

The computer-readable storage device may include various devices capable of storing program codes, such as a Universal Serial Bus flash drive, a mobile hard disk, a Read-Only Memory (ROM), a Random Access Memory (RAM), a magnetic disk, or an optical disc.

### Second embodiment

In some examples, in order to determine the human physiological state under the control of the electroencephalogram signal, a physiological state prediction model may be used to predict the human physiological state based on the electroencephalogram signal. The electroencephalogram signal may be obtained based on an electroencephalogram (EEG). The physiological state prediction model includes, for example, a deep learning model. Specifically, an end-to-end deep learning model may be used for physiological state prediction. Although the end-to-end deep learning model may automatically learn some features with good discriminability, it often also fits to some non-important features, causing the model to locally collapse to some poor features. As a result, the model has low accuracy in predicting physiological state and poor prediction performance.

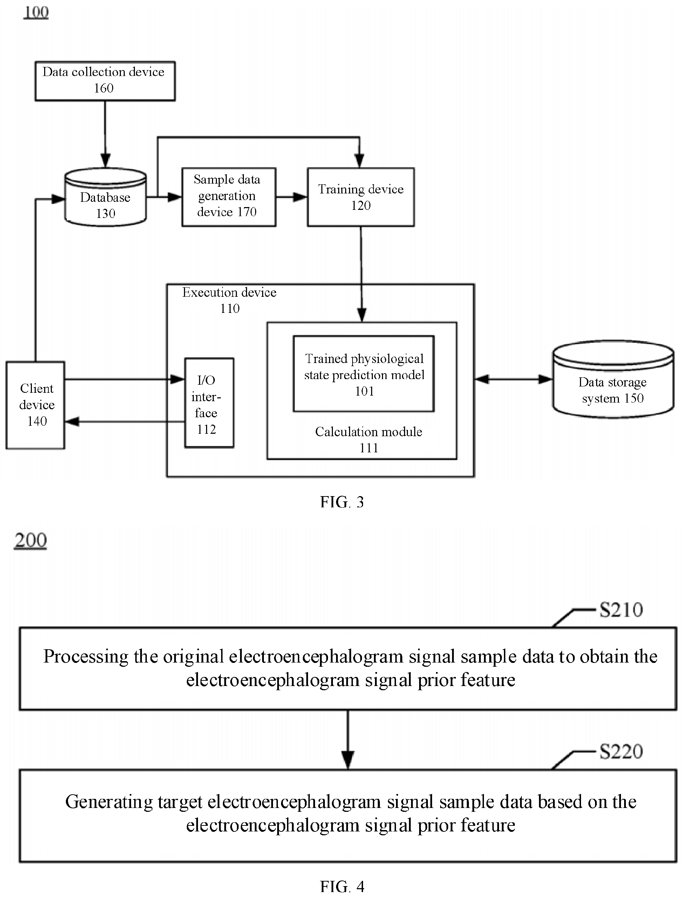

In view of this, the implementations of the present disclosure provide an optimized method for generating electroencephalogram signal sample data, a training method for a physiological state prediction model, and a physiological state prediction method. A system architecture shown in FIG. 3 may be configured to implement at least one of the method for generating the electroencephalogram signal sample data, the training method for the physiological state prediction model, or the physiological state prediction method. As shown in FIG. 3, the system architecture 100 includes an execution device 110, a training device 120, a database 130, a client device 140, a data storage system 150, a data collection device 160, and a sample data generation device 170. The execution device 110 includes a computing module 111 and an I/O interface 112, and a trained physiological state prediction model 101 is stored in the computing module 111.

The data collection device 160 is configured to collect original electroencephalogram signal sample data and store the collected data in the database 130.

The sample data generation device 170 performs data processing on the original electroencephalogram signal sample data stored in the database 130 to obtain an electroencephalogram signal prior feature, and generates target electroencephalogram signal sample data based on the electroencephalogram signal prior feature.

A to-be-trained physiological state prediction model is stored in the training device 120. The training device 120 performs auxiliary training on the to-be-trained physiological state prediction model based on the target electroencephalogram signal sample data. Generally, the training adopts a deep learning method, where auxiliary learning, transfer learning, reinforcement learning, and manifold learning involve adding additional network branches during the training stage (i.e., the training phase), to better complete the task of training the neural network model. The auxiliary training mentioned in the present disclosure adopts an auxiliary learning training method, and an auxiliary physiological state prediction model may be obtained after the auxiliary training. The training device 120 also trains the auxiliary physiological state prediction model based on the original electroencephalogram signal sample data stored in the database 130 to obtain a trained physiological state prediction model 101. The trained physiological state prediction model 101 obtained by the training device 120 may be applied to different systems or devices, such as the execution device 110.

The execution device 110 is configured with an I/O interface 112, and performs data interaction with external devices through the I/O interface 112. The external devices include, for example, the client device 140. A user may input original electroencephalogram signal data into the I/O interface 112 through the client device 140. The original electroencephalogram signal data may be collected by the data collection device 160, stored in the database 130, and then inputted into the execution device 110 after user confirmation or corresponding processing. In addition, the user may also input an instruction through the client device 140 into the I/O interface 112 to instruct the execution device 110 to perform a data processing operation.

The execution device 110 may call data, codes, and the like in the data storage system 150, and may also store data, instructions, and the like in the data storage system 150.

The computing module 111 stores the trained physiological state prediction model 101. The computing module 111 uses the trained physiological state prediction model 101 to predict and obtain a physiological state prediction result based on the inputted original electroencephalogram signal data.

The prior feature data mentioned in the present disclosure is obtained based on the original electroencephalogram signal sample data, i.e., in general, the prior feature may be extracted from the original electroencephalogram signal sample data.

Subsequently, the I/O interface 112 may return the prediction result to the client device 140 for the user.

In the scenario shown in FIG. 1, the user may manually specify the original electroencephalogram signal data inputted to the execution device 110, for example, by performing an input operation on an interface provided by the I/O interface 112. In another scenario, the client device 140 may automatically input the original electroencephalogram signal sample data into the I/O interface 112. For example, the client device 140 automatically inputs the original electroencephalogram signal sample data after user's authorization is obtained, and the user may set a corresponding permission in the client device 140. The user may view the physiological state prediction result outputted by the execution device 110 on the client device 140, and the specific presentation form may be display, sound, action, or other specific forms. The client device 140 may also store the predicted physiological state prediction result in the database 130.

It's worth noting that FIG. 3 is only a schematic diagram of a system architecture provided by an embodiment of the present disclosure, and a positional relationship between devices, components, modules, and the like shown in the figure does not constitute any limitation. For example, in FIG. 1, the data storage system 150 is an external memory relative to the execution device 110. In other cases, the data storage system 150 may also be placed in the execution device 110.

FIG. 4 is a schematic flowchart of a method for generating electroencephalogram signal sample data provided by an implementation of the present disclosure.

As shown in FIG. 4, the method 200 for generating the electroencephalogram signal sample data provided by the implementation of the present disclosure includes, for example, steps S210 to S220.

At step S210, the original electroencephalogram signal sample data is processed to obtain the electroencephalogram signal prior feature.

Exemplarily, the original electroencephalogram signal sample data may be electroencephalogram signal data collected based on a brain-computer interface, or data obtained after the collected electroencephalogram signal data is preprocessed. The preprocessing includes operations such as performing band-pass filtering on the collected electroencephalogram signal data and removing redundant data. The performing band-pass filtering on the collected electroencephalogram signal data includes removing information with an excessively high frequency or an excessively low frequency in the electroencephalogram signal data.

Exemplarily, the electroencephalogram signal prior feature is prior knowledge relative to the original electroencephalogram signal sample data. Generally, the electroencephalogram signal prior feature includes important information or high discriminative information in the original electroencephalogram signal sample data. Adding the prior knowledge into a model training process may optimize model's performance, reduce model's training difficulty, and improve a model's generalization capability during the training.

At step S220, target electroencephalogram signal sample data is generated based on the electroencephalogram signal prior feature.

Exemplarily, the target electroencephalogram signal sample data is used for the auxiliary training of the to-be-trained physiological state prediction model, to obtain the auxiliary physiological state prediction model. The to-be-trained physiological state prediction model includes, for example, the deep learning model, and specifically may include a Convolutional Neural Networks (CNN) model or other models based on the CNN model.

After the auxiliary physiological state prediction model is obtained through training, the auxiliary physiological state prediction model may be further trained based on the original electroencephalogram signal sample data to obtain a trained physiological state prediction model. The trained physiological state prediction model may be configured to predict physiological states that include a motor imagery state, an emotional state, and the like.

In the embodiment of the present disclosure, the electroencephalogram signal prior feature is obtained based on the original electroencephalogram signal sample data, and the target electroencephalogram signal sample data is generated based on the electroencephalogram signal prior feature. Since the target electroencephalogram signal sample data contains prior information, performing the auxiliary training on the to-be-trained physiological state prediction model based on the target electroencephalogram signal sample data can improve prediction accuracy of the model. After the auxiliary physiological state prediction model is obtained through training, the training continues to be performed on the auxiliary physiological state prediction model based on the original electroencephalogram signal sample data to obtain the trained physiological state prediction model, which further improves the prediction accuracy of the model. It can be seen that adding the electroencephalogram signal prior feature for auxiliary training during model training can optimize the model's performance, reduce the model's training difficulty, improve the model's generalization capability, and improve the model's prediction accuracy.

It can be understood that the physiological state prediction model in the second embodiment of the present disclosure has the same function and implementation as the electroencephalogram expert subsystem in the first embodiment as described above. Training the physiological state prediction model in the second embodiment may also be regarded as training the electroencephalogram expert subsystem, i.e., the physiological state prediction model in the second embodiment may be replaced with the electroencephalogram expert subsystem. In addition, in this case, the electroencephalogram training set used for training the electroencephalogram expert subsystem equivalently includes the original electroencephalogram signal sample data and the target electroencephalogram signal sample data.

Next, the process of generating target electroencephalogram signal sample data based on the electroencephalogram signal prior feature will be further elaborated. The target electroencephalogram signal sample data includes first target electroencephalogram signal sample data and second target electroencephalogram signal sample data.

In an example, the first target electroencephalogram signal sample data may be constructed based on the electroencephalogram signal prior feature. For example, the electroencephalogram signal prior feature may be directly used as the first target electroencephalogram signal sample data. Alternatively, in a case where a data format of the first target electroencephalogram signal sample data is predefined as a predetermined data format, the electroencephalogram signal prior feature may be decomposed or transformed, or otherwise processed according to the predetermined data format, to construct the first target electroencephalogram signal sample data. It can be understood that directly constructing the first target electroencephalogram signal sample data for auxiliary model training based on the electroencephalogram signal prior feature allows the model to focus on prior knowledge that is beneficial for classification, thereby improving the targeted processing ability of the auxiliary physiological state prediction model's ability to target important information.

In another example, the original electroencephalogram signal sample data and the electroencephalogram signal prior feature may be merged to obtain the second target electroencephalogram signal sample data. For example, data decomposition and fusion may be performed on the original electroencephalogram signal sample data and the electroencephalogram signal prior feature to obtain the second target electroencephalogram signal sample data. Alternatively, data concatenation may be performed on the original electroencephalogram signal sample data and the electroencephalogram signal prior feature to obtain the second target electroencephalogram signal sample data. For example, when the original electroencephalogram signal sample data is vector data or matrix data, the electroencephalogram signal prior feature may be concatenated at a specific position in the vector data or matrix data, and the specific position includes a head position, a tail position, or any intermediate position. In some cases, preferably selecting the tail position as the specific position may improve a model's training effect and model's prediction accuracy. It can be understood that merging the original electroencephalogram signal sample data with the electroencephalogram signal prior feature to obtain the second target electroencephalogram signal sample data for the auxiliary model training allows the model to take into account both the prior knowledge and the original data, thereby improving the generalization capability of the auxiliary physiological state prediction model.

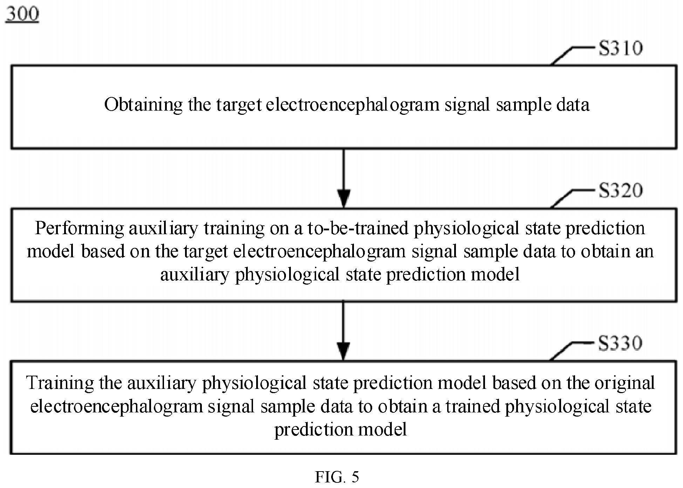

FIG. 5 is a schematic flowchart of a training method for a physiological state prediction model provided by an implementation of the present disclosure.

As shown in FIG. 5, the training method 300 for the physiological state prediction model provided by the implementation of the present disclosure includes, for example, steps S310 to S330.

At step S310, the target electroencephalogram signal sample data is obtained.

Exemplarily, the target electroencephalogram signal sample data is determined based on an electroencephalogram signal prior feature, and the electroencephalogram signal prior feature is obtained based on the original electroencephalogram signal sample data.

At step S320, auxiliary training is performed on the to-be-trained physiological state prediction model based on the target electroencephalogram signal sample data to obtain an auxiliary physiological state prediction model.

At step S330, the auxiliary physiological state prediction model is trained based on the original electroencephalogram signal sample data to obtain a trained physiological state prediction model.

It can be understood that in the embodiments of the present disclosure, the electroencephalogram signal prior feature is obtained based on the original electroencephalogram signal sample data, and the target electroencephalogram signal sample data is obtained based on the electroencephalogram signal prior feature. Since the target electroencephalogram signal sample data contains the prior information, performing the auxiliary training on the to-be-trained physiological state prediction model based on the target electroencephalogram signal sample data improves the prediction accuracy of the model. After the auxiliary physiological state prediction model is obtained through training, the auxiliary physiological state prediction model continues to be trained based on the original electroencephalogram signal sample data to obtain the trained physiological state prediction model, which further improves the prediction accuracy of the model. In addition, the embodiments of the present disclosure may directly use the original electroencephalogram signal sample data as the input of the model, which avoids the cumbersome process of sample data preprocess and manual feature selection, and saves time and labor costs.

The embodiments of the present disclosure perform two trainings on the model based on an auxiliary learning method. The model is trained based on the auxiliary learning method, which allows the model to learn more detailed classification weights and makes the model pay more attention to a feature that is beneficial to classification. In the first training, the auxiliary physiological state prediction model is obtained through auxiliary training based on the target electroencephalogram signal sample data with prior knowledge. The first training enables the model to obtain the ability to predict the physiological state based on the prior knowledge. After the auxiliary physiological state prediction model is obtained, the auxiliary physiological state prediction model is directly transferred for the second training. In the second training, the auxiliary physiological state prediction model is trained based on the original electroencephalogram signal sample data. Since the auxiliary physiological state prediction model has undergone the first training, a relevant parameter of the model can be finely adjusted in the second training, which further improves the prediction accuracy of the model while improving the training efficiency.

Next, the process of obtaining the target electroencephalogram signal sample data will be further elaborated. The target electroencephalogram signal sample data includes the first target electroencephalogram signal sample data and the second target electroencephalogram signal sample data.

In an example, the electroencephalogram signal prior feature may be obtained by processing the original electroencephalogram signal sample data, and then the first target electroencephalogram signal sample data is constructed according to the electroencephalogram signal prior feature. For example, the electroencephalogram signal prior feature may be directly used as the first target electroencephalogram signal sample data, or relevant processing such as decomposition or transformation may be performed on the electroencephalogram signal prior feature according to the predetermined data format that is predefined to construct the first target electroencephalogram signal sample data. For details, reference can be made to the above description, and details are omitted herein. It can be understood that the first target electroencephalogram signal sample data for auxiliary model training is directly constructed based on the electroencephalogram signal prior feature and model training is performed on the first target electroencephalogram signal sample data, which makes the model focus on the prior knowledge that is beneficial to classification, and improves the auxiliary physiological state prediction model's ability to target important information.

In another example, the electroencephalogram signal prior feature may be obtained by processing the original electroencephalogram signal sample data, and then the original electroencephalogram signal sample data and the electroencephalogram signal prior feature are merged to obtain the second target electroencephalogram signal sample data. For example, the data decomposition and fusion may be performed on the original electroencephalogram signal sample data and the electroencephalogram signal prior feature to obtain the second target electroencephalogram signal sample data. Alternatively, the data concatenation may be performed on the original electroencephalogram signal sample data and the electroencephalogram signal prior feature to obtain the second target electroencephalogram signal sample data. For details, reference can be made to the above description, and details are omitted herein. It can be understood that merging the original electroencephalogram signal sample data with the electroencephalogram signal prior feature to obtain the second target electroencephalogram signal sample data for auxiliary model training and performing the model training allow the model to take into account both the prior knowledge and the original data, improving the generalization ability of the auxiliary physiological state prediction model. The data concatenation is performed on the original electroencephalogram signal sample data and the electroencephalogram signal prior feature to train a classification model with a set number of labels (for example, predetermined labels of the prior feature include four types: left hand, right hand, happy, and sad, and the corresponding classifications of original electroencephalogram signal samples include two labels: motor imagery and emotion). After the data concatenation, deep training may be performed on the six-classification training model. The labels are marked corresponding to different physiological states during physiological signal collection, such as 0, 1, 2, 3, 4, 5, etc., corresponding to different physiological states.

In another embodiment of the present disclosure, an appropriate electroencephalogram signal prior feature for model training may be determined through feature engineering.

For example, the original electroencephalogram signal sample data may be processed according to a predetermined feature indicator to obtain a feature value corresponding to the predetermined feature indicator as the electroencephalogram signal prior feature.

The predetermined feature indicator may include, for example: total energy of (δ+θ+α+β), absolute energy of δ wave, absolute energy of θ wave, absolute energy of α wave, absolute energy of β wave, Renyi entropy, Wavelet Transform, Wavelet Absolute Mean, FFT Mean Coefficient, minimum value indicator, maximum value indicator, average value indicator, variance indicator, standard deviation indicator, differential median indicator, maximum spectral peak indicator, and the like. The predetermined feature indicator may be set according to actual situations, and is not exhaustively listed here. As an example, the minimum value indicator is used as the predetermined feature indicator. The original electroencephalogram signal sample data is processed to obtain the minimum value of the original electroencephalogram signal sample data, and the minimum value is a feature value corresponding to the minimum value indicator. As an example, the maximum spectral peak indicator is used as the predetermined feature indicator. The original electroencephalogram signal sample data is transformed from the time domain to the frequency domain to obtain a frequency spectrum, and the maximum spectral peak is calculated as a feature value corresponding to the maximum spectral peak indicator. Then, the minimum value and the maximum spectral peak are used as the electroencephalogram signal prior feature.

Exemplarily, an appropriate predetermined feature indicator may be determined through feature engineering. For example, a plurality to-be-verified feature indicator groups are determined from a plurality of candidate feature indicators, and each to-be-verified feature indicator group includes at least one candidate feature indicator. The plurality of candidate feature indicators include, for example, a minimum value indicator, a maximum value indicator, an average value indicator, a variance indicator, a standard deviation indicator, a differential median indicator, a maximum spectral peak indicator, and the like. As an example, a first to-be-verified feature indicator group includes 10 feature indicators such as a minimum value indicator, a maximum value indicator, and an average value indicator. Similarly, a second of to-be-verified feature indicator group includes 10 feature indicators such as an average value indicator, a variance indicator, and a standard deviation indicator, and a third to-be-verified feature indicator group includes 10 feature indicators such as a standard deviation indicator, a differential median indicator, and a maximum spectral peak indicator.

After the plurality of to-be-verified feature indicator groups are obtained, the plurality of to-be-verified feature indicator groups are verified respectively, to obtain a verification result corresponding to each to-be-verified feature indicator group. The verification result represents a prediction accuracy degree of a physiological state prediction model for indicator verification when the physiological state prediction model predicts the physiological state based on feature values corresponding to each to-be-verified feature indicator group. The physiological state prediction model for indicator verification may include the above to-be-trained physiological state prediction model, the above auxiliary physiological state prediction model, the above trained physiological state prediction model, or other physiological state prediction models.

Then, based on the verification result, at least one group corresponding to high model prediction accuracy is selected from the plurality of to-be-verified feature indicator groups, and preferably a group corresponding to the highest model prediction accuracy is selected. A feature indicator included in the selected to-be-verified feature indicator group is used as the predetermined feature indicator. The indicator verification process may be completed before the model training. After the predetermined feature indicator is obtained through the indicator verification process, the electroencephalogram signal prior feature is obtained by performing data processing on the original electroencephalogram signal sample data based on the predetermined feature indicator. Alternatively, the indicator verification process may also be performed simultaneously with the model training process. For example, a plurality of rounds of model training may be performed, and each round of model training is performed based on an electroencephalogram signal prior feature obtained from different predetermined feature indicators. Finally, at least one to-be-verified feature indicator group with the best training effect is used as the predetermined feature indicator.

Next, training processes for a to-be-predicted physiological state prediction model and the auxiliary physiological state prediction model will be further elaborated.

For the training process for the to-be-predicted physiological state prediction model, in an example, in the case where the first target electroencephalogram signal sample data is constructed based on the electroencephalogram signal prior feature, when the to-be-trained physiological state prediction model is trained, the first target electroencephalogram signal sample data may be inputted into the to-be-trained physiological state prediction model for prediction to obtain a first fine-grained classification prediction result representing a physiological state. Then, based on the first fine-grained classification prediction result, a model parameter of the to-be-trained physiological state prediction model is adjusted to obtain the auxiliary physiological state prediction model. For example, the first target electroencephalogram signal sample data includes a first sample label, and the model parameter of the to-be-trained physiological state prediction model may be adjusted based on a loss value between the first fine-grained classification prediction result and the first sample label to obtain the auxiliary physiological state prediction model.

Exemplarily, the first target electroencephalogram signal sample data is used to control physiological states, and the physiological states include broad categories such as a motor imagery state and an emotional state. The motor imagery state may be further subdivided into fine categories such as a left-hand movement state and a right-hand movement state. The emotional state may be further subdivided into fine categories such as a happy state and a sad state. The first target electroencephalogram signal sample data is used to control hand movements and emotions. The hand movements include a left-hand movement and a right-hand movement, and the emotions include happiness and sadness. Therefore, the motor imagery state and the emotional state may be predicted based on the first target electroencephalogram signal sample data. It can be understood that except for the motor imagery state and the emotional state, the physiological states may also include other physiological states set according to actual needs. Except for the left-hand movement state and the right-hand movement state, the motor imagery state may also include other motor imagery states set according to actual needs. Except for the happy state and the sad state, the emotional state may also include other emotional states set according to actual needs.

The to-be-trained physiological state prediction model is, for example, a four-classification model that is used to perform the physiological state prediction based on the first target electroencephalogram signal sample data to obtain the first fine-grained classification prediction result. The first fine-grained classification prediction result includes, for example, a left-hand movement state, a right-hand movement state, a happy state, and a sad state. Specifically, a probability of left-hand movement state, a probability of right-hand movement state, a probability of happy state, and a probability of sad state can be outputted.

For the training process for the to-be-predicted physiological state prediction model, in another example, in the case where the original electroencephalogram signal sample data and the electroencephalogram signal prior feature are merged to obtain the second target electroencephalogram signal sample data, when the to-be-trained physiological state prediction model is trained, the second target electroencephalogram signal sample data is inputted into the to-be-trained physiological state prediction model for prediction to obtain a second fine-grained classification prediction result and a first coarse-grained classification prediction result that represent a physiological state. Then, based on the second fine-grained classification prediction result and the first coarse-grained classification prediction result, a model parameter of the to-be-trained physiological state prediction model is adjusted to obtain the auxiliary physiological state prediction model. For example, the second target electroencephalogram signal sample data includes a second sample label, and the model parameter of the to-be-trained physiological state prediction model may be adjusted based on loss values between both the second fine-grained classification prediction result and the first coarse-grained classification prediction result and the second sample label to obtain the auxiliary physiological state prediction model.

Exemplarily, the to-be-trained physiological state prediction model is, for example, a six-classification model that is used to perform the physiological state prediction based on the second target electroencephalogram signal sample data to obtain the second fine-grained classification prediction result and the first coarse-grained classification prediction result. The second fine-grained classification prediction result includes, for example, a left-hand movement state, a right-hand movement state, a happy state, and a sad state. The first coarse-grained classification prediction result includes a motor imagery state and an emotional state. Specifically, the probability of left-hand movement state, the probability of right-hand movement state, the probability of happy state, the probability of sad state, the probability of motor imagery state, and the probability of emotional state can be outputted.

For the training process for the auxiliary physiological state prediction model, after the auxiliary physiological state prediction model is obtained through auxiliary training, the original electroencephalogram signal sample data is further inputted into the auxiliary physiological state prediction model for prediction to obtain a second coarse-grained classification prediction result representing the physiological state. Then, based on the second coarse-grained classification prediction result, a model parameter of the auxiliary physiological state prediction model is adjusted to obtain the trained physiological state prediction model. For example, the original electroencephalogram signal sample data includes a third sample label, and the model parameter of the auxiliary physiological state prediction model may be adjusted based on a loss value between the second coarse-grained classification prediction result and the third sample label to obtain the trained physiological state prediction model.

Exemplarily, the original electroencephalogram signal sample data includes, for example, electroencephalogram signal data for controlling the occurrence of left-hand movement state, electroencephalogram signal data for controlling the occurrence of right-hand movement state, electroencephalogram signal data for controlling the occurrence of happy state, and electroencephalogram signal data for controlling the occurrence of sad state. The auxiliary physiological state prediction model trained based on the first target electroencephalogram signal sample data is, for example, a four-classification model, and the auxiliary physiological state prediction model trained based on the second target electroencephalogram signal sample data is, for example, a six-classification model. The auxiliary physiological state prediction model has the capability of processing the electroencephalogram signal data for controlling the occurrence of left-hand movement state, the occurrence of right-hand movement state, the occurrence of happy state, and the occurrence of sad state. When a second round of model training is performed, a four-classification or six-classification auxiliary physiological state prediction model may be set as a two-classification model that is used to output two classification results. The two-classification auxiliary physiological state prediction model is used to perform the physiological state prediction based on the original electroencephalogram signal sample data to obtain the second coarse-grained classification prediction result. The second coarse-grained classification prediction result includes, for example, the motor imagery state and the emotional state. Specifically, the probability of motor imagery state and the probability of emotional state can be outputted.

It can be understood that the electroencephalogram signal prior feature contains important information or highly discriminative information, so the electroencephalogram signal prior feature is more accurate for predicting the fine categories of physiological states. After the first target electroencephalogram signal sample data is constructed based on the electroencephalogram signal prior feature, using the first target electroencephalogram signal sample data to train the auxiliary physiological state prediction model for performing fine-grained classification on the physiological states can improve fine-grained classification prediction accuracy of the model. After the auxiliary physiological state prediction model for fine-grained classification is obtained, on this basis, a physiological state prediction model for performing coarse-grained classification on the physiological states is trained based on the original electroencephalogram signal sample data, which enables the coarse-grained classification to be performed on the basis of the fine-grained classification, thereby improving accuracy of the coarse-grained classification.

It can be understood that after the second target electroencephalogram signal sample data is obtained by merging the original electroencephalogram signal sample data and the electroencephalogram signal prior feature, the second target electroencephalogram signal sample data is used to train an auxiliary physiological state prediction model for both fine-grained classification and coarse-grained classification simultaneously. By considering coarse-grained classification on the basis of fine-grained classification, the model not only has a relatively accurate fine-grained classification capability, but also has a preliminary coarse-grained classification capability. On this basis, the physiological state prediction model for coarse-grained classification is further trained based on the original electroencephalogram signal sample data, which further strengthens the coarse-grained classification ability of the model and improves the accuracy of coarse-grained classification of the model.

It can be understood that the embodiments of the present disclosure provide a method for inputting the prior knowledge into the model for training. It is feasible to input the combination of the prior knowledge and the original electroencephalogram signal sample data into the model, which further proves that performing the model training based on the auxiliary learning method is feasible and the model obtained through training has a good prediction effect. The model after auxiliary training based on the electroencephalogram signal prior feature has a good classification effect in recognizing the motor imagery state and the emotional state.

In order to facilitate the understanding of the training process for the physiological state prediction model of the present disclosure, FIG. 4 takes the physiological state prediction model as a convolutional neural network model for illustration. It can be understood that the model structure shown in FIG. 4 and examples of a data size or a data dimension given below for easy understanding are for reference only, and the present disclosure does not impose specific restrictions on the model structure, the data size, or the data dimension.

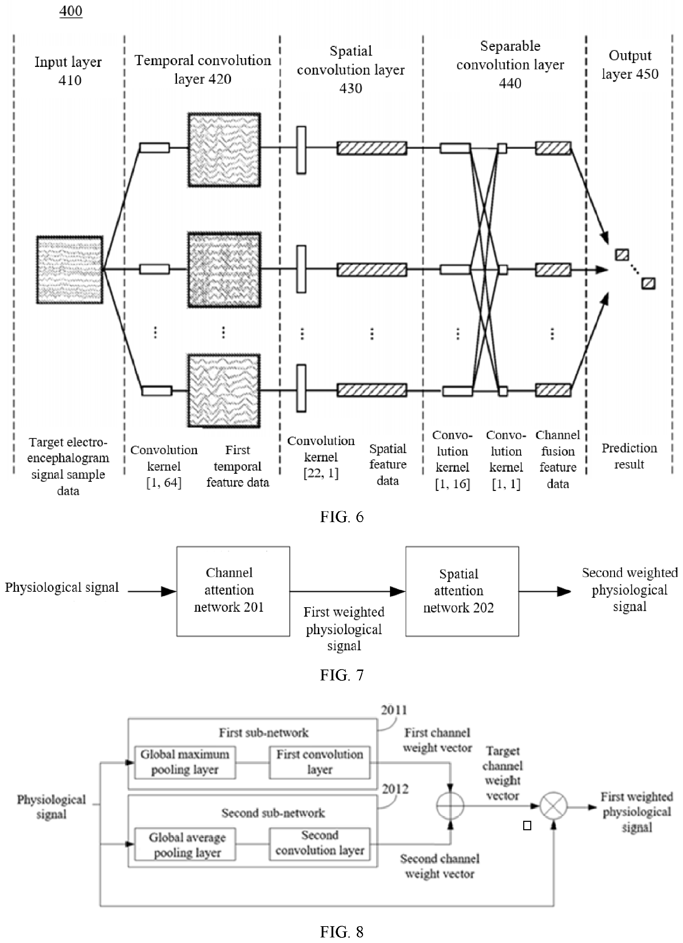

FIG. 6 is a schematic diagram of a physiological state prediction model provided by an implementation of the present disclosure.

As shown in FIG. 6, the physiological state prediction model 400 may be a to-be-trained physiological state prediction model or an auxiliary physiological state prediction model. Unlike other models that are typically only applicable to physiological state prediction for a single Brain Computer Interface (BCI) paradigm, the physiological state prediction model 400 of the embodiments of the present disclosure may be applied to physiological state prediction for a plurality of BCI paradigms. The BCI paradigm refers to a method for performing a data collection experiment based on a BCI. For example, the process of collecting the electroencephalogram signal corresponding to the motor imagery state is one paradigm, and the process of collecting the electroencephalogram signal corresponding to the emotional state is another paradigm. The physiological state prediction model 400 at least includes a temporal convolution layer 420, a spatial convolution layer 430, and a separable convolution layer 440 that are connected in sequence. Of course, the physiological state prediction model 400 may also include an input layer 410 and an output layer 450.

When the physiological state prediction model 400 is the to-be-trained physiological state prediction model, the temporal convolution layer 420 is configured to extract first temporal feature data from the target electroencephalogram signal sample data (including the first target electroencephalogram signal sample data and the second target electroencephalogram signal sample data) along a time dimension. When the physiological state prediction model 400 is the auxiliary physiological state prediction model, the temporal convolution layer 420 is configured to extract first temporal feature data from the original electroencephalogram signal sample data along the time dimension. The spatial convolution layer 430 is configured to extract spatial feature data from the first temporal feature data along a spatial dimension. The separable convolution layer 440 is configured to extract second temporal feature data from the spatial feature data along the time dimension and to perform feature fusion on the second temporal feature data.

An exemplary process of the model training will be described below with examples.

First, an electroencephalogram signal of a subject may be collected multiple times under different physiological states to obtain the original electroencephalogram signal sample data. For example, one piece of original electroencephalogram signal sample data may be collected through one experiment, and may include the electroencephalogram signal data corresponding to the left-hand movement state, the right-hand movement state, the happy state, and the sad state. The collected data may also be preprocessed, and the preprocessed data may be used as the original electroencephalogram signal sample data. The preprocessing includes operations such as performing band-pass filtering on the collected electroencephalogram signal data and removing redundant data.

Then, the electroencephalogram signal prior feature is obtained based on the original electroencephalogram signal sample data, and the target electroencephalogram signal sample data (including the first target electroencephalogram signal sample data and the second target electroencephalogram signal sample data) is obtained based on the electroencephalogram signal prior feature. The target electroencephalogram signal sample data may be divided into a training set, a validation set, and a test set. For example, one piece of target electroencephalogram signal sample data includes electroencephalogram signal data collected over 10 seconds. The 10-second electroencephalogram signal data is divided into three segments: 8-second data segment, 1-second data segment, and 1-second data segment, respectively. The 8-second data segment is used as the training set, the 1-second data segment is used as the validation set to verify whether the model is overfitting or underfitting, and the other 1-second data is used as the test set to evaluate the prediction accuracy of the trained model. The physiological state prediction model 400 of the embodiments of the present disclosure includes a compact deep learning model. After the model is trained based on the training set of the target electroencephalogram signal sample data (including the first target electroencephalogram signal sample data and the second target electroencephalogram signal sample data) or the original electroencephalogram signal sample data, the trained model is used to classify the validation set and the test set, enabling the model to accurately classify the physiological states.

During the model training, as an example, the original electroencephalogram signal sample data has a data size of [22, 990]. Taking the second target electroencephalogram signal sample data as an example, the original electroencephalogram signal sample data and the electroencephalogram signal prior feature are merged to obtain the second target electroencephalogram signal sample data with a data size such as [22, 1000], where 22 denotes the number of channels. The number of channels is the number of electrodes that are configured to collect the original electroencephalogram signal sample data. For example, each channel collects 990 data points over 10 seconds.

First, the second target electroencephalogram signal sample data is inputted into the model through the input layer 410.

Second, feature extraction is performed on the second target electroencephalogram signal sample data through the temporal convolution layer 420. For example, the feature extraction is performed using a convolution kernel with a data size of [1, 64], and then F1 filters (not shown) are used to extract F1 types of time dimension information, to obtain first temporal feature data with a data size of [F1, 22, 1000] along the time dimension, where F1 is a predetermined integer value.

Then, the first temporal feature data is inputted into the spatial convolution layer 430 for feature extraction. For example, the spatial convolution layer 430 performs spatial feature extraction using a convolution kernel with a data size of [22, 1], and then is connected to D*F1 spatial filters (not shown) to extract spatial dimension information. The extracted spatial feature data has a data size such as [D*F1, 1, 1000], where D is a predetermined integer value.

Next, the spatial feature data is inputted into the separable convolution layer 440 to learn temporal features of each channel and fuse or integrate data of the plurality of channels. The separable convolution layer 440 includes, for example, a temporal convolution kernel with a data size of [1, 16] and an ordinary convolution kernel with a data size of [1, 1]. The temporal convolution kernel with a data size of [1, 16] can be used to perform the feature extraction on the spatial feature data to obtain second temporal feature data for each channel, and the ordinary convolution kernel with a data size of [1, 1] can be used to perform the feature fusion on the second temporal feature data of the plurality of channels, to realize the fusion or integration of the feature data of the plurality of channels, obtaining channel-fused feature data for the plurality of channels.

Finally, the feature data outputted by the separable convolution layer 440 is transmitted to the output layer 450, and a physiological state prediction result is outputted through the output layer 450. The output layer 450 may include a fully connected layer that classifies the learned deep features to predict the current physiological state.

It can be understood that the physiological state prediction model extracts the temporal feature, the spatial feature, and the channel-fused feature through the temporal convolution layer, the spatial convolution layer, and the separable convolution layer, which improves the diversity of features and allows the extracted features to contain deep-level important information, thereby improving the prediction accuracy of the physiological state prediction model.

Through the embodiments of the present disclosure, the physiological state prediction model learns a weight of the motor imagery state and a weight of the emotional state, and performs physiological state classification through the fully connected layer. Based on the auxiliary learning method, after the model is trained on more refined subcategories such as a left-hand movement state, a right-hand movement state, a happy state, and a sad state, the model is more accurate and reliable in the recognition of large categories such as a motor imagery state and an emotional state.

In one implementation, the physiological state prediction method 500 provided by the implementations of the present disclosure includes, for example, the following steps.

At a first step, original electroencephalogram signal data is obtained.

At a second step, the original electroencephalogram signal data is inputted into a trained physiological state prediction model for prediction, to obtain a physiological state prediction result.

The trained physiological state prediction model may be obtained through the training method for the physiological state prediction model as described above, and details are omitted herein. The predicted physiological state prediction result includes, for example, a motor imagery state and an emotional state, and specifically may be the probability of the motor imagery state and the probability of the emotional state. Since the trained physiological state prediction model is trained based on the prior knowledge of the electroencephalogram signal, using the trained physiological state prediction model to perform the physiological state prediction based on the original electroencephalogram signal data allows for higher accuracy of the prediction result.

In one implementation, another physiological state prediction method 600 provided by the implementations of the present disclosure includes, for example, the following steps.

At a first step, original electroencephalogram signal data is obtained.

At a second step, the original electroencephalogram signal data is inputted into a trained physiological state prediction model for prediction, to obtain a physiological state prediction result.

The trained physiological state prediction model includes at least one of a trained first physiological state prediction model or a trained second physiological state prediction model. For example, one model with higher prediction accuracy may be selected from the trained first physiological state prediction model and the trained second physiological state prediction model to predict the original electroencephalogram signal data.

The trained first physiological state prediction model is obtained by training based on the first target electroencephalogram signal sample data. The trained second physiological state prediction model is obtained by training based on the second target electroencephalogram signal sample data. The first target electroencephalogram signal sample data is constructed based on the electroencephalogram signal prior feature that is obtained by processing the original electroencephalogram signal sample data. The second target electroencephalogram signal sample data is obtained by merging the original electroencephalogram signal sample data with the electroencephalogram signal prior feature.

Exemplarily, the trained first physiological state prediction model is obtained through the following steps: performing auxiliary training on the to-be-trained first physiological state prediction model based on the first target electroencephalogram signal sample data to obtain a first auxiliary physiological state prediction model; and training the first auxiliary physiological state prediction model based on the original electroencephalogram signal sample data to obtain the trained first physiological state prediction model. The model training process can refer to the above content, and details are omitted herein.

Exemplarily, the trained second physiological state prediction model is obtained through the following steps: performing auxiliary training on the to-be-trained second physiological state prediction model based on the second target electroencephalogram signal sample data to obtain a second auxiliary physiological state prediction model; and training the second auxiliary physiological state prediction model based on the original electroencephalogram signal sample data to obtain the trained second physiological state prediction model. The model training process can refer to the above content, and details are omitted herein.

An apparatus for generating electroencephalogram signal sample data provided by the implementations of the present disclosure includes: a processing module and a generation module.

Exemplarily, the processing module is configured to process original electroencephalogram signal sample data to obtain an electroencephalogram signal prior feature.

Exemplarily, the generation module is configured to generate target electroencephalogram signal sample data based on the electroencephalogram signal prior feature. The target electroencephalogram signal sample data is used for auxiliary training of a to-be-trained physiological state prediction model to obtain an auxiliary physiological state prediction model. The original electroencephalogram signal sample data is used to train the auxiliary physiological state prediction model to obtain a trained physiological state prediction model.

Exemplarily, the generation module is at least configured to perform at least one of: constructing first target electroencephalogram signal sample data based on the electroencephalogram signal prior feature; or merging the original electroencephalogram signal sample data with the electroencephalogram signal prior feature to obtain second target electroencephalogram signal sample data.

Exemplarily, the processing module is specifically configured to process the original electroencephalogram signal sample data according to a predetermined feature indicator to obtain a feature value corresponding to the predetermined feature indicator as the electroencephalogram signal prior feature.

Exemplarily, the apparatus for generating the electroencephalogram signal sample data further includes a first model training module configured to: input the first target electroencephalogram signal sample data into the to-be-trained physiological state prediction model for prediction to obtain a first fine-grained classification result representing a physiological state; and adjust a model parameter of the to-be-trained physiological state prediction model based on the first fine-grained classification result to obtain the auxiliary physiological state prediction model.

Exemplarily, the first fine-grained classification result includes at least one of a left-hand movement state, a right-hand movement state, a happy state, or a sad state.

Exemplarily, the first model training module is further configured to: input the second target electroencephalogram signal sample data into the to-be-trained physiological state prediction model for prediction to obtain a second fine-grained classification result and a first coarse-grained classification result that represent a physiological state; and adjust a model parameter of the to-be-trained physiological state prediction model based on the second fine-grained classification result and the first coarse-grained classification result to obtain the auxiliary physiological state prediction model.

Exemplarily, the second fine-grained classification result includes at least one of: a left-hand movement state, a right-hand movement state, a happy state, or a sad state. The first coarse-grained classification result includes at least one of: a motor imagery state or an emotional state.

Exemplarily, the first model training module is further configured to: input the original electroencephalogram signal sample data into the auxiliary physiological state prediction model for prediction to obtain a second coarse-grained classification result representing a physiological state; and adjust a model parameter of the auxiliary physiological state prediction model based on the second coarse-grained classification result to obtain the trained physiological state prediction model.

Exemplarily, the second coarse-grained classification result includes at least one of: a motor imagery state or an emotional state.

Exemplarily, the original electroencephalogram signal sample data includes at least one of: electroencephalogram signal data for controlling the occurrence of left-hand movement state, electroencephalogram signal data for controlling the occurrence of right-hand movement state, electroencephalogram signal data for controlling the occurrence of happy state, or electroencephalogram signal data for controlling the occurrence of sad state.

Exemplarily, the processing module is further configured to: determine a plurality of to-be-verified feature indicator groups from a plurality of candidate feature indicators, where each of the plurality of to-be-verified feature indicator groups includes at least one candidate feature indicator; respectively verify each of the plurality of to-be-verified feature indicator groups to obtain a verification result corresponding to each of the plurality of to-be-verified feature indicator groups, where the verification result represents the prediction accuracy of the physiological state prediction model used for indicator verification when the physiological state prediction is performed based on the feature value corresponding to each of the plurality of to-be-verified feature indicator groups; and select at least one to-be-verified feature indicator group from the plurality of to-be-verified feature indicator groups based on the verification result as the predetermined feature indicator.

Exemplarily, the to-be-trained physiological state prediction model or the auxiliary physiological state prediction model includes: a temporal convolution layer configured to extract first temporal feature data from the target electroencephalogram signal sample data or the original electroencephalogram signal sample data along a time dimension; a spatial convolution layer configured to extract spatial feature data from the first temporal feature data along a spatial dimension; and a separable convolution layer configured to extract second temporal feature data from the spatial feature data along the time dimension and perform feature fusion on the second temporal feature data.

It can be understood that a specific implementation process of the apparatus for generating the electroencephalogram signal sample data can refer to the implementation process of the method for generating the electroencephalogram signal sample data as described above, and details are omitted herein.

A training apparatus for a physiological state prediction model provided by the implementations of the present disclosure includes: a first obtaining module, a first training module, and a second training module.

Exemplarily, the first obtaining module is configured to obtain target electroencephalogram signal sample data. The target electroencephalogram signal sample data is determined based on an electroencephalogram signal prior feature, and the electroencephalogram signal prior feature is obtained based on original electroencephalogram signal sample data.

Exemplarily, the first training module is configured to perform auxiliary training on a to-be-trained physiological state prediction model based on the target electroencephalogram signal sample data to obtain an auxiliary physiological state prediction model.

Exemplarily, the second training module is configured to train the auxiliary physiological state prediction model based on the original electroencephalogram signal sample data to obtain a trained physiological state prediction model.

Exemplarily, the first obtaining module is specifically configured to: process the original electroencephalogram signal sample data to obtain an electroencephalogram signal prior feature; and construct first target electroencephalogram signal sample data based on the electroencephalogram signal prior feature.

Exemplarily, the first obtaining module is specifically configured to: process the original electroencephalogram signal sample data to obtain an electroencephalogram signal prior feature; and merging the original electroencephalogram signal sample data with the electroencephalogram signal prior feature to obtain second target electroencephalogram signal sample data.

Exemplarily, said processing the original electroencephalogram signal sample data to obtain the electroencephalogram signal prior feature includes: processing the original electroencephalogram signal sample data according to a predetermined feature indicator to obtain a feature value corresponding to the predetermined feature indicator as the electroencephalogram signal prior feature.

Exemplarily, the first training module is specifically configured to: input the first target electroencephalogram signal sample data into the to-be-trained physiological state prediction model for prediction to obtain a first fine-grained classification result representing a physiological state; and adjust a model parameter of the to-be-trained physiological state prediction model based on the first fine-grained classification result to obtain the auxiliary physiological state prediction model.

Exemplarily, the first fine-grained classification result includes at least one of: a left-hand movement state, a right-hand movement state, a happy state, or a sad state.

Exemplarily, the first training module is specifically configured to: input the second target electroencephalogram signal sample data into the to-be-trained physiological state prediction model for prediction to obtain a second fine-grained classification result and a first coarse-grained classification result that represent a physiological state; and adjust a model parameter of the to-be-trained physiological state prediction model based on the second fine-grained classification result and the first coarse-grained classification result to obtain the auxiliary physiological state prediction model.

Exemplarily, the second fine-grained classification result includes at least one of: a left-hand movement state, a right-hand movement state, a happy state, or a sad state. The first coarse-grained classification result includes at least one of: a motor imagery state or an emotional state.

Exemplarily, the second training module is specifically configured to: input the original electroencephalogram signal sample data into the auxiliary physiological state prediction model for prediction to obtain a second coarse-grained classification result representing a physiological state; and adjust a model parameter of the auxiliary physiological state prediction model based on the second coarse-grained classification result to obtain the trained physiological state prediction model.

Exemplarily, the second coarse-grained classification result includes at least one of: a motor imagery state or an emotional state.

Exemplarily, the original electroencephalogram signal sample data includes at least one of: electroencephalogram signal data for controlling the occurrence of left-hand movement state, electroencephalogram signal data for controlling the occurrence of right-hand movement state, electroencephalogram signal data for controlling the occurrence of happy state, or electroencephalogram signal data for controlling the occurrence of sad state.

Exemplarily, the predetermined feature indicator is obtained by: determining a plurality of to-be-verified feature indicator groups from a plurality of candidate feature indicators, where each of the plurality of to-be-verified feature indicator groups includes at least one candidate feature indicator; respectively verifying each of the plurality of to-be-verified feature indicator groups to obtain a verification result corresponding to each of the plurality of to-be-verified feature indicator groups, where the verification result represents the prediction accuracy of the physiological state prediction model for indicator verification when the physiological state prediction is performed based on the feature value corresponding to each of the plurality of to-be-verified feature indicator groups; and selecting at least one to-be-verified feature indicator group from the plurality of to-be-verified feature indicator groups based on the verification result as the predetermined feature indicator.

Exemplarily, the to-be-trained physiological state prediction model or the auxiliary physiological state prediction model includes: a temporal convolution layer configured to extract first temporal feature data from the target electroencephalogram signal sample data or the original electroencephalogram signal sample data along a time dimension; a spatial convolution layer configured to extract spatial feature data from the first temporal feature data along a spatial dimension; and a separable convolution layer configured to extract second temporal feature data from the spatial feature data along the time dimension and to perform feature fusion on the second temporal feature data.

It can be understood that a specific implementation process of the training apparatus for the physiological state prediction model can refer to the implementation process of the training method for the physiological state prediction model as described above, and details are omitted herein.

A physiological state prediction apparatus provided by the implementations of the present disclosure includes: a second obtaining module and a first prediction module.

Exemplarily, the second obtaining module is configured to obtain original electroencephalogram signal data.

Exemplarily, the first prediction module is configured to input the original electroencephalogram signal data into a trained physiological state prediction model for prediction to obtain a physiological state prediction result.

Exemplarily, the physiological state prediction apparatus further includes a second model training module. The second model training module is specifically configured to: obtain target electroencephalogram signal sample data, where the target electroencephalogram signal sample data is determined based on an electroencephalogram signal prior feature, and the electroencephalogram signal prior feature is obtained based on the original electroencephalogram signal sample data; perform auxiliary training on the to-be-trained physiological state prediction model based on the target electroencephalogram signal sample data to obtain an auxiliary physiological state prediction model; and train the auxiliary physiological state prediction model based on the original electroencephalogram signal sample data to obtain a trained physiological state prediction model.

Exemplarily, the physiological state prediction apparatus further includes a first sample obtaining module. The first sample obtaining module is configured to: process the original electroencephalogram signal sample data to obtain the electroencephalogram signal prior feature; and construct first target electroencephalogram signal sample data based on the electroencephalogram signal prior feature.

Exemplarily, the first sample obtaining module is further configured to: process the original electroencephalogram signal sample data to obtain the electroencephalogram signal prior feature; and merge the original electroencephalogram signal sample data with the electroencephalogram signal prior feature to obtain second target electroencephalogram signal sample data.

Exemplarily, the first sample obtaining module is further configured to: process the original electroencephalogram signal sample data according to a predetermined feature indicator to obtain a feature value corresponding to the predetermined feature indicator as the electroencephalogram signal prior feature.

Exemplarily, the second model training module is further configured to: input the first target electroencephalogram signal sample data into the to-be-trained physiological state prediction model for prediction to obtain a first fine-grained classification result representing a physiological state; and adjust a model parameter of the to-be-trained physiological state prediction model based on the first fine-grained classification result to obtain the auxiliary physiological state prediction model.

Exemplarily, the first fine-grained classification result includes at least one of: a left-hand movement state, a right-hand movement state, a happy state, or a sad state.

Exemplarily, the second model training module is further configured to: input the second target electroencephalogram signal sample data into the to-be-trained physiological state prediction model for prediction to obtain a second fine-grained classification result and a first coarse-grained classification result that represent the physiological state; and adjust a model parameter of the to-be-trained physiological state prediction model based on the second fine-grained classification result and the first coarse-grained classification result to obtain the auxiliary physiological state prediction model.

Exemplarily, the second fine-grained classification result includes at least one of: a left-hand movement state, a right-hand movement state, a happy state, or a sad state. The first coarse-grained classification result includes at least one of: a motor imagery state or an emotional state.

Exemplarily, the second model training module is further configured to: input the original electroencephalogram signal sample data into the auxiliary physiological state prediction model for prediction to obtain a second coarse-grained classification result representing a physiological state; and adjust a model parameter of the auxiliary physiological state prediction model based on the second coarse-grained classification result to obtain the trained physiological state prediction model.

Exemplarily, the second coarse-grained classification result includes at least one of: a motor imagery state or an emotional state.

Exemplarily, the original electroencephalogram signal sample data includes at least one of: electroencephalogram signal data for controlling the occurrence of left-hand movement state, electroencephalogram signal data for controlling the occurrence of right-hand movement state, electroencephalogram signal data for controlling the occurrence of happy state, or electroencephalogram signal data for controlling the occurrence of sad state.

Exemplarily, the first sample obtaining module is further configured to: determine a plurality of to-be-verified feature indicator groups from a plurality of candidate feature indicators, where each of the plurality of to-be-verified feature indicator group includes at least one candidate feature indicator; respectively verify each of the plurality of to-be-verified feature indicator groups to obtain a verification result corresponding to each of the plurality of to-be-verified feature indicator groups, where the verification result represents the prediction accuracy of the physiological state prediction model for indicator verification when the physiological state prediction is performed based on feature values corresponding to each of the plurality of to-be-verified feature indicator groups; and select at least one group of to-be-verified feature indicators from the plurality of to-be-verified feature indicator groups based on the verification result as the predetermined feature indicators.

Exemplarily, the to-be-trained physiological state prediction model or the auxiliary physiological state prediction model includes: a temporal convolution layer configured to extract first temporal feature data from the target electroencephalogram signal sample data or the original electroencephalogram signal sample data along a time dimension; a spatial convolution layer configured to extract spatial feature data from the first temporal feature data along a spatial dimension; and a separable convolution layer configured to extract second temporal feature data from the spatial feature data along the time dimension and to perform feature fusion on the second temporal feature data.

It can be understood that a specific implementation process of the physiological state prediction apparatus can refer to the implementation process of the physiological state prediction method as described above, and details are omitted herein.

Another physiological state prediction apparatus provided by the implementations of the present disclosure includes: a third obtaining module and a second prediction module.

Exemplarily, the third obtaining module is configured to obtain original electroencephalogram signal data.

Exemplarily, the second prediction module is configured to input the original electroencephalogram signal data into a trained physiological state prediction model for prediction, to obtain a physiological state prediction result. The trained physiological state prediction model includes a trained first physiological state prediction model and a trained second physiological state prediction model. The trained first physiological state prediction model is obtained by training based on first target electroencephalogram signal sample data. The trained second physiological state prediction model is obtained by training based on second target electroencephalogram signal sample data. The first target electroencephalogram signal sample data is constructed based on an electroencephalogram signal prior feature, and the electroencephalogram signal prior feature is obtained by processing the original electroencephalogram signal sample data. The second target electroencephalogram signal sample data is obtained by merging the original electroencephalogram signal sample data with the electroencephalogram signal prior feature.

Exemplarily, another physiological state prediction apparatus further includes a first physiological state prediction model training module. The first physiological state prediction model training module is configured to: perform auxiliary training on a to-be-trained first physiological state prediction model based on the first target electroencephalogram signal sample data to obtain a first auxiliary physiological state prediction model; and train the first auxiliary physiological state prediction model based on the original electroencephalogram signal sample data to obtain a trained first physiological state prediction model.

Exemplarily, another physiological state prediction apparatus further includes a second physiological state prediction model training module. The second physiological state prediction model training module is configured to: perform auxiliary training on a to-be-trained second physiological state prediction model based on the second target electroencephalogram signal sample data to obtain a second auxiliary physiological state prediction model; and train the second auxiliary physiological state prediction model based on the original electroencephalogram signal sample data to obtain a trained second physiological state prediction model.

Exemplarily, another physiological state prediction apparatus further includes a second sample obtaining module. The second sample obtaining module is configured to: process the original electroencephalogram signal sample data to obtain an electroencephalogram signal prior feature; and construct the first target electroencephalogram signal sample data according to the electroencephalogram signal prior feature.

Exemplarily, the second sample obtaining module is further configured to: process the original electroencephalogram signal sample data to obtain the electroencephalogram signal prior feature; and merge the original electroencephalogram signal sample data with the electroencephalogram signal prior feature to obtain the second target electroencephalogram signal sample data.

Exemplarily, the second sample obtaining module is configured to: process the original electroencephalogram signal sample data according to a predetermined feature indicator to obtain a feature value corresponding to the predetermined feature indicator as the electroencephalogram signal prior feature.

Exemplarily, the first physiological state prediction model training module is further configured to: input the first target electroencephalogram signal sample data into the to-be-trained first physiological state prediction model for prediction to obtain a first fine-grained classification prediction result representing a physiological state; and adjust a model parameter of the to-be-trained first physiological state prediction model based on the first fine-grained classification prediction result to obtain the first auxiliary physiological state prediction model.

Exemplarily, the first fine-grained classification prediction result includes at least one of: a left-hand movement state, a right-hand movement state, a happy state, or a sad state.

Exemplarily, the second physiological state prediction model training module is further configured to: input the second target electroencephalogram signal sample data into the to-be-trained second physiological state prediction model for prediction to obtain a second fine-grained classification prediction result and a first coarse-grained classification prediction result that represent the physiological state; and adjust a model parameter of the to-be-trained second physiological state prediction model based on the second fine-grained classification prediction result and the first coarse-grained classification prediction result to obtain the second auxiliary physiological state prediction model.

Exemplarily, the second fine-grained classification prediction result includes at least one of: a left-hand movement state, a right-hand movement state, a happy state, or a sad state. The first coarse-grained classification prediction result includes at least one of: a motor imagery state or an emotional state.

Exemplarily, the first physiological state prediction model training module or the second physiological state prediction model training module is further configured to: input the original electroencephalogram signal sample data into the first auxiliary physiological state prediction model or the second auxiliary physiological state prediction model for prediction to obtain a second coarse-grained classification prediction result representing a physiological state; and adjust a model parameter of the first auxiliary physiological state prediction model or the second auxiliary physiological state prediction model based on the second coarse-grained classification prediction result to obtain the trained first physiological state prediction model or the trained second physiological state prediction model.

Exemplarily, the second coarse-grained classification prediction result includes at least one of: a motor imagery state or an emotional state.

Exemplarily, the original electroencephalogram signal sample data includes at least one of: electroencephalogram signal data for controlling the occurrence of left-hand movement state, electroencephalogram signal data for controlling the occurrence of right-hand movement state, electroencephalogram signal data for controlling the occurrence of happy state, or electroencephalogram signal data for controlling the occurrence of sad state.

Exemplarily, the second sample obtaining module is further configured to: determine a plurality of to-be-verified feature indicator group from a plurality of candidate feature indicators, where each of the plurality of to-be-verified feature indicator groups includes at least one candidate feature indicator; respectively verify each of the plurality of to-be-verified feature indicator groups to obtain a verification result corresponding to each of the plurality of to-be-verified feature indicator groups, where the verification result represents the prediction accuracy of the physiological state prediction model for indicator verification when the physiological state prediction is performed based on feature values corresponding to each of the plurality of to-be-verified feature indicator groups; and select at least one to-be-verified feature indicator group from the plurality of to-be-verified feature indicator groups based on the verification result as the predetermined feature indicator.

Exemplarily, the to-be-trained first physiological state prediction model, the to-be-trained second physiological state prediction model, the first auxiliary physiological state prediction model, or the second auxiliary physiological state prediction model includes: a temporal convolution layer, a spatial convolution layer, and a separable convolution layer. The temporal convolution layer of the to-be-trained first physiological state prediction model is configured to extract first temporal feature data from the first target electroencephalogram signal sample data along a temporal dimension. The temporal convolution layer of the to-be-trained second physiological state prediction model is configured to extract first temporal feature data from the second target electroencephalogram signal sample data along the temporal dimension. The first auxiliary physiological state prediction model or the second auxiliary physiological state prediction model is configured to extract first temporal feature data from the original electroencephalogram signal sample data along the temporal dimension. The spatial convolution layer is configured to extract spatial feature data from the first temporal feature data along a spatial dimension. The separable convolution layer is configured to extract second temporal feature data from the spatial feature data along the temporal dimension and to perform feature fusion on the second temporal feature data.

It can be understood that a specific implementation process of another physiological state prediction apparatus can refer to the implementation process of another physiological state prediction method as described above, and details are omitted herein.

### Third embodiment

At present, users may monitor their health states using a physiological collection device combined with an analysis device. For example, the physiological collection device may collect a physiological signal of a user, upload the physiological signal to the analysis device, and the analysis device may then analyze and process the physiological signal to obtain the user's health state and feed this state back to the user. The physiological collection device may be configured to collect at least one of the following physiological signals: an electroencephalographic signal, a respiratory signal, a heart rate signal, an electrodermal activity signal, a skin temperature signal, an electromyographic signal, or the like.

Since different types of physiological signals require different processing methods, after receiving the physiological signal uploaded by the physiological collection device, the analysis device first needs to recognize the type of the physiological signal before accurately analyzing the user's health state based on this type.

For the first embodiment of the present disclosure, in which a classification task needs to be performed by a classification model for multimodal human physiological data, the data is used to monitor one of the health states via the physiological collection device combined with the analysis device. Therefore, before the classification task, the type of physiological data may also be recognized according to the technical solution in this embodiment. In addition, the physiological signal in this embodiment also refers to the physiological data in the first embodiment of the present disclosure.

This embodiment of the present disclosure provides a human-factor intelligence-based physiological signal processing method, which is applied to an electronic device. In one implementation, the electronic device may be a terminal device or a server. The server may be a single server, a server cluster composed of a plurality of servers, or a cloud computing service center. The method includes the following steps.

At a first step, weighting processing is performed on an inputted physiological signal through a channel attention network to obtain a first weighted physiological signal.

During the processing of the physiological signal by the channel attention network, the number of channels of the physiological signal is not compressed, i.e., the number of channels of the physiological signal does not change in this process. In this way, it can be ensured that the channel attention network can reduce its computational complexity while capturing cross-channel interaction information during the processing of the physiological signal by the channel attention network.

The physiological signal may be transmitted to the electronic device by the physiological collection device, or may be pre-stored in the electronic device. In one implementation, the physiological signal may be a single type of signal. For example, the physiological signal may be an electroencephalogram signal, an electromyography signal, or an electrodermal activity signal. Alternatively, the physiological signal may include various types of signals. For example, the physiological signal may include an electroencephalogram signal, an electromyography signal, and an electrodermal activity signal.

When processing the physiological signal through the channel attention network to obtain the first weighted physiological signal, the electronic device may first determine a target channel weight vector of the physiological signal, and then perform weighting processing on the physiological signal based on the target channel weight vector to obtain the first weighted physiological signal.

At a second step, weighting processing is performed on the first weighted physiological signal through a spatial attention network to obtain a second weighted physiological signal.

After obtaining the first weighted physiological signal, the electronic device may input the first weighted physiological signal into the spatial attention network, so that the spatial attention network performs the weighting processing on the first weighted physiological signal, thereby obtaining the second weighted physiological signal.

It can be understood that during the processing of the physiological signal through the spatial attention network to obtain the second weighted physiological signal, the electronic device may first determine a target spatial weight vector, and then perform the weighting processing on the first weighted physiological signal based on the target spatial weight vector, thereby obtaining the first weighted physiological signal.

At a third step, the type of the physiological signal is obtained based on the second weighted physiological signal.

In the embodiment of the present disclosure, the electronic device may input the physiological signal into a physiological signal recognition network to obtain the type of the physiological signal. The type may include electroencephalogram, electromyography, electrocardiogram, electrodermal activity, skin temperature, blood flow, or the like.

To sum up, the embodiments of the present disclosure provide a human-factor intelligence-based physiological signal processing method. This method may perform the weighting processing on the physiological signal through the channel attention network to obtain the first weighted physiological signal, and perform the weighting processing on the first weighted physiological signal through the spatial attention network to obtain the second weighted physiological signal, and then recognize and obtain the type of the physiological signal based on the second weighted physiological signal. Since the number of channels of the physiological signal is not compressed during the processing of the physiological signal by the channel attention network, the computational complexity of the channel attention network is reduced, thereby improving recognition efficiency of the type of the physiological signal.

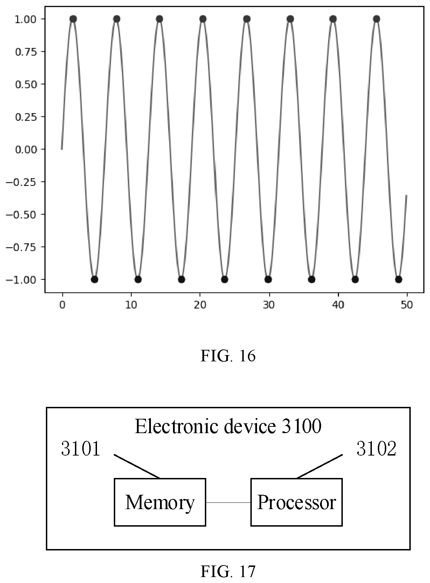

In the embodiments of the present disclosure, the electronic device is equipped with a recognition model, and may process the physiological signal through the recognition model to obtain the type of the physiological signal. FIG. 7 shows a schematic structural diagram of a recognition model. Referring to FIG. 7, the recognition model includes a channel attention network 201 and a spatial attention network 202 that are connected in sequence. Referring to FIG. 16, the channel attention network includes: a first sub-network 2011 and a second sub-network 2012 that are in parallel. The process of the electronic device executing the first step may include the following steps.

At step S1, the physiological signal is inputted into the first sub-network to obtain a first channel weight vector.

During the processing of the physiological signal by the first sub-network, the number of channels of the physiological signal is not compressed, i.e., the number of channels of the physiological signal does not change in the process where the first sub-network processes the physiological signal. Since the number of channels is not compressed, there is no need to perform an operation to restore the number of channels, which can reduce the computational complexity of the first sub-network in the process of determining the first channel weight vector.

In the embodiment of the present disclosure, continue to refer to FIG. 8, the first sub-network includes: a global max pooling layer and a first convolution layer that are connected in sequence. The electronic device may compress the physiological signal along a spatial dimension through the global max pooling layer to obtain a first compressed physiological signal. Then, the electronic device may perform convolution processing on the first compressed physiological signal through the first convolution layer to obtain the first channel weight vector of the physiological signal. In one implementation, the first convolution layer is a one-dimensional convolution layer.

In the embodiments of the present disclosure, after obtaining the first compressed physiological signal, the electronic device may use the transpose() function to transpose the first compressed physiological signal, to enable the first convolution layer to perform the convolution processing on the first compressed physiological signal. Then, after obtaining an output result of the first convolution layer, the electronic device may use the transpose() function again to perform transpose the output result, thereby obtaining the first channel weight vector.

At step S2, the physiological signal is inputted into the second sub-network to obtain a second channel weight vector.

During the processing of the physiological signal by the second sub-network, the number of channels of the physiological signal is not compressed, i.e., the number of channels of the physiological signal does not change in the process where the second sub-network processes the physiological signal. Since the number of channels is not compressed, there is no need to perform the operation to restore the number of channels, which can reduce the computational complexity of the second sub-network in the process of determining the second channel weight vector.

As shown in FIG. 8, the second sub-network 2012 may include: a global average pooling layer and a second convolution layer that are connected in sequence. The electronic device may compress the physiological signal inputted to the channel attention network along the spatial dimension through the global average pooling layer to obtain a second compressed physiological signal. Then, the electronic device may perform convolution processing on the second compressed physiological signal through the second convolution layer to obtain the second channel weight vector.

It can be understood that after obtaining the second compressed physiological signal, the electronic device may use the transpose() function to transpose the second compressed physiological signal, to enable the second convolution layer to perform the convolution processing on the first compressed physiological signal. Then, after obtaining an output result of the second convolution layer, the electronic device may use the transpose() function again to transpose the output result, thereby obtaining the second channel weight vector.

At step S3, the first weighted physiological signal is obtained based on the first channel weight vector, the second channel weight vector, and the physiological signal.

In the embodiments of the present disclosure, as shown in FIG. 8, the electronic device may perform weighted summation on the first channel weight vector and the second channel weight vector to obtain a target channel weight vector. Then, the electronic device may perform the weighting processing on the physiological signal using the target channel weight vector to obtain the first weighted physiological signal. For example, the electronic device may multiply the target channel weight vector by the physiological signal to obtain the first weighted physiological signal.

It can be understood that both a weight for the first channel weight vector and a weight for the second channel weight vector may be pre-stored in the electronic device. For example, both the weight for the first channel weight vector and the weight for the second channel weight vector may be 1. At this time, the electronic device may perform an element-wise summation operation on the first channel weight vector and the second channel weight vector, and then may apply an activation function to the resulting weight vector obtained after summation, thereby obtaining the target channel weight vector.

The activation function may be the sigmoid() function.

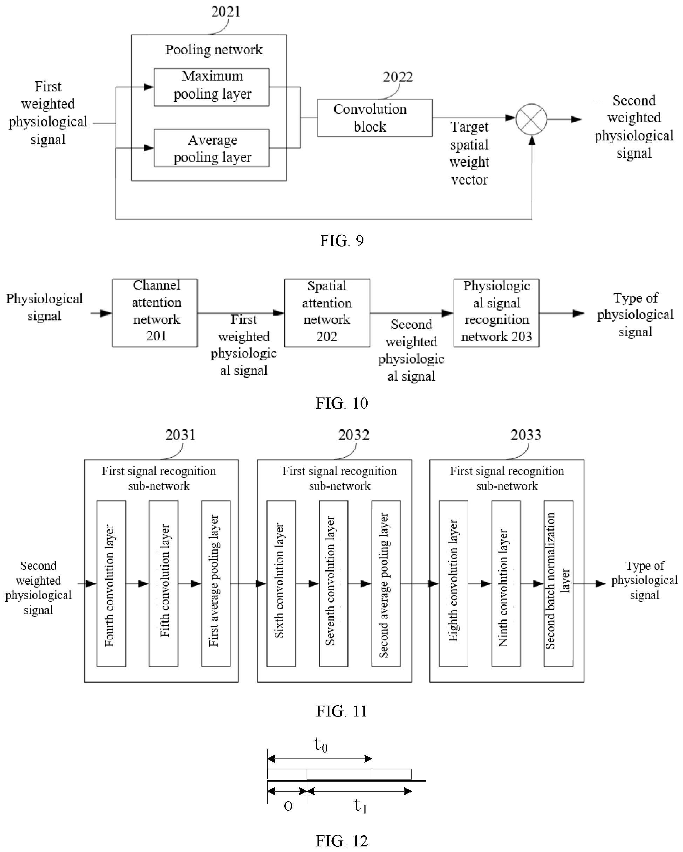

FIG. 9 is a schematic diagram of a structure of a spatial attention network provided by an embodiment of the present disclosure. As can be seen from FIG. 9, the spatial attention network 202 may include: a pooling network 2021 and a convolution block 2022. Based on this, the process of the electronic device processing the first weighted physiological signal through the spatial attention network to obtain the second weighted physiological signal may include the following steps.

At step A1, the first weighted physiological signal is compressed along a channel dimension based on the pooling network to obtain a third compressed physiological signal.

Continue to refer to FIG. 9, the pooling network 2021 may include: a max pooling layer and an average pooling layer. The electronic device may compress the first weighted physiological signal along the channel dimension through the max pooling layer to obtain a first sub-compressed physiological signal, and compress the first weighted physiological signal along the channel dimension through the average pooling layer to obtain a second sub-compressed physiological signal. Then, the electronic device may concatenate the first sub-compressed physiological signal and the second sub-compressed physiological signal to obtain a third compressed physiological signal.

For example, the electronic device may use the concat() function to concatenate the first sub-compressed physiological signal and the second sub-compressed physiological signal to obtain the third compressed physiological signal.

At step A2, convolution processing is performed on the third compressed physiological signal through the convolution block to obtain the target spatial weight vector.

In the embodiments of the present disclosure, the convolution block may include: a third convolution layer and a first batch normalization (BN) layer that are connected in sequence. The electronic device may perform the convolution processing on the third compressed physiological signal through the third convolution layer to obtain an initial spatial weight vector. Then, the electronic device may perform normalization processing on the initial spatial weight vector through the first BN layer to obtain the normalized initial spatial weight vector. After that, the electronic device may apply an activation function to perform an operation on the batch-normalized initial spatial weight vector to obtain the target spatial weight vector.

Since the convolution block is used for signal processing in the spatial attention network, problems of overfitting and gradient vanishing can be effectively reduced.

It can be understood that the activation function may be the mish() function.

At step A3, weighting processing is performed on the first weighted physiological signal using the target spatial weight vector to obtain the second weighted physiological signal.

After obtaining the target spatial weight vector, the electronic device may use the target spatial weight vector to perform the weighting processing on the first weighted physiological signal to obtain the second weighted physiological signal. For example, the electronic device may multiply the first weighted physiological signal by the target spatial weight vector to obtain the second weighted physiological signal.

FIG. 10 is a schematic diagram of a structure of another recognition model provided by an embodiment of the present disclosure. Referring to FIG. 10, the model may further include: a physiological signal recognition network 203 connected to the spatial attention network 202. The electronic device may input the second weighted physiological signal into the physiological signal recognition network to obtain the type of the physiological signal. In one implementation, the physiological signal recognition network may be a convolutional neural network.

Referring to FIG. 11, the physiological signal recognition network 203 may include: a first signal recognition sub-network 2031, a second signal recognition sub-network 2032, and a third signal recognition sub-network 2033 that are connected in sequence. The first signal recognition sub-network 2031 includes: a fourth convolution layer, a fifth convolution layer, and a first average pooling layer that are connected in sequence. The second signal recognition sub-network 2032 includes: a sixth convolution layer, a seventh convolution layer, and a second average pooling layer that are connected in sequence. The third signal recognition sub-network 2033 includes: an eighth convolution layer, a ninth convolution layer, and a second BN layer that are connected in sequence.

A size of a convolution kernel of the fourth convolution layer is different from a size of a convolution kernel of the fifth convolution layer. For example, the size of the convolution kernel of the fourth convolution layer may be 3*3, and the size of the convolution kernel of the fifth convolution layer may be 1*1. A size of a convolution kernel of the sixth convolution layer is different from a size of a convolution kernel of the seventh convolution layer. For example, the size of the convolution kernel of the sixth convolution layer may be 1*4, and the size of the convolution kernel of the seventh convolution layer may be 1*1. A size of a convolution kernel of the first average pooling layer is different from a size of a convolution kernel of the second average pooling layer. For example, the size of the convolution kernel of the first average pooling layer may be 2*4, and the size of the convolution kernel of the second average pooling layer may be 2*2. A size of a convolution kernel of the eighth convolution layer is different from a size of a convolution kernel of the ninth convolution layer. For example, the size of the convolution kernel of the eighth convolution layer may be 2*2, and the size of the convolution kernel of the ninth convolution layer may be 1*1.

The electronic device may perform the convolution processing on the second weighted physiological signal sequentially through the fourth convolution layer and the fifth convolution layer to obtain a first processing result. Then, the electronic device inputs the first processing result into the first average pooling layer. The first average pooling layer may compress the first processing result to obtain a second processing result.

Then, the electronic device may perform the convolution processing on the second processing result sequentially through the sixth convolution layer and the seventh convolution layer to obtain a third processing result. Then, the electronic device inputs the third processing result into the second average pooling layer. The second average pooling layer may compress the third processing result to obtain a fourth processing result.

After that, the electronic device may perform the convolution processing on the fourth processing result sequentially through the eighth convolution layer and the ninth convolution layer to obtain a fifth processing result, and input the fifth processing result into the second BN layer. The second BN layer may perform the normalization processing on the fifth processing result to obtain the type of the physiological signal.

It can be understood that the sequence of steps of the human-factor intelligence-based physiological signal processing method provided in the embodiments of the present disclosure may be appropriately adjusted, and the steps may be added or deleted as need. Any method easily conceived by those skilled in the art within the technical scope disclosed in the present disclosure shall fall within the scope of the present disclosure, and details are omitted herein.

To sum up, the embodiments of the present disclosure provide a human-factor intelligence-based physiological signal processing method. This method may perform the weighting processing on the physiological signal through the channel attention network to obtain the first weighted physiological signal, perform the weighting processing on the first weighted physiological signal through the spatial attention network to obtain the second weighted physiological signal, and then recognize and obtain the type of the physiological signal based on the second weighted physiological signal. Since the number of channels of the physiological signal is not compressed during the processing of the physiological signal by the channel attention network, the computational complexity of the channel attention network is reduced, thereby improving the recognition efficiency of the type of the physiological signal.

The embodiments of the present disclosure further provide a human-factor intelligence-based physiological signal processing apparatus. The apparatus includes: a first weighting processing module configured to perform weighting processing on an inputted physiological signal through a channel attention network to obtain a first weighted physiological signal, where the number of channels of the physiological signal is not compressed during the processing of the physiological signal by the channel attention network; a second weighting processing module configured to perform weighting processing on the first weighted physiological signal through a spatial attention network to obtain a second weighted physiological signal; and a recognition module configured to obtain a type of the physiological signal based on the second weighted physiological signal.

In one implementation, the channel attention network includes: a first sub-network and a second sub-network. The first weighting processing module may be configured to: input the physiological signal into the first sub-network to obtain a first channel weight vector, where the number of channels of the physiological signal is not compressed during the processing of the physiological signal by the first sub-network; input the physiological signal into the second sub-network to obtain a second channel weight vector, where the number of channels of the physiological signal is not compressed during the processing of the physiological signal by the second sub-network; and obtain the first weighted physiological signal based on the first channel weight vector, the second channel weight vector, and the physiological signal.

In one implementation, the first sub-network includes: a global max pooling layer and a first convolution layer that are connected in sequence. The first weighting processing module may be configured to: compress the physiological signal along the spatial dimension through the global max pooling layer to obtain a first compressed physiological signal; and perform convolution processing on the first compressed physiological signal through the first convolution layer to obtain the first channel weight vector of the physiological signal.

In one implementation, the second sub-network includes: a global average pooling layer and a second convolution layer that are connected in sequence. The first weighting processing module may be configured to: compress the physiological signal along the spatial dimension through the global average pooling layer to obtain a second compressed physiological signal; and perform convolution processing on the second compressed physiological signal through the second convolution layer to obtain the second channel weight vector of the physiological signal.

In one implementation, the first weighting processing module may be configured to: perform weighted summation on the first channel weight vector and the second channel weight vector to obtain a target channel weight vector; and perform weighting processing on the physiological signal using the target channel weight vector to obtain the first weighted physiological signal.

In one implementation, the spatial attention network includes: a pooling network and a convolution block. The second weighting processing module may be configured to: compress the first weighted physiological signal along the channel dimension based on the pooling network to obtain a third compressed physiological signal; perform convolution processing on the third compressed physiological signal through the convolution block to obtain a target spatial weight vector; and perform weighting processing on the first weighted physiological signal using the target spatial weight vector to obtain the second weighted physiological signal.

In one implementation, the pooling network includes: a max pooling layer and an average pooling layer. The second weighting processing module may be configured to: compress the first weighted physiological signal along the channel dimension through the max pooling layer to obtain a first sub-compressed physiological signal; compress the first weighted physiological signal along the channel dimension through the average pooling layer to obtain a second sub-compressed physiological signal; and concatenate the first sub-compressed physiological signal and the second sub-compressed physiological signal to obtain a third compressed physiological signal.

In one implementation, the recognition module may be configured to: process the second weighted physiological signal through a physiological signal recognition network to obtain a type of the physiological signal.

To sum up, the embodiments of the present disclosure provide a human-factor intelligence-based physiological signal processing apparatus. The apparatus may perform the weighting processing on the physiological signal through the channel attention network to obtain the first weighted physiological signal, perform the weighting processing on the first weighted physiological signal through the spatial attention network to obtain the second weighted physiological signal, and then recognize and obtain the type of the physiological signal based on the second weighted physiological signal. Since the number of channels of the physiological signal is not compressed during the processing of the physiological signal by the channel attention network, the computational complexity of the channel attention network is reduced, thereby improving the recognition efficiency of the type of the physiological signal.

### Fourth embodiment

A human-factor intelligence-based physiological signal processing method and apparatus, a HRV feature extraction method and apparatus, an electronic device, and a computer-readable storage medium provided in the embodiments of the present disclosure are described below with reference to the accompanying drawings.

A human-factor intelligence-based physiological signal processing method provided in the embodiments of the present disclosure may include the following steps.

At a first step, an original physiological signal is obtained, and slicing processing is performed on the original physiological signal to obtain a plurality of physiological signal segments.

In order to extract more accurate features from the original physiological signal and provide a more accurate judgment basis for medical diagnosis, purification processing may be performed on the original physiological signal to remove noise from the original physiological signal. However, if the original physiological signal is too long, a purification effect may not be optimal. Therefore, the slicing processing can be performed on the original physiological signal, and then the purification processing can be performed on sliced physiological signal segments.

In the embodiments of the present disclosure, the original physiological signal may include an electrocardiogram signal and an electroencephalogram signal. For example, the original physiological signal may be the electrocardiogram (ECG) signal or the electroencephalogram (EEG) signal.

According to an embodiment of the present disclosure, specific implementation of performing the slicing processing on the original physiological signal may include: segmenting the original physiological signal using a time window. At least adjacent time windows overlap. Moreover, each time window remains a consistent duration, i.e., each time window has the same time length .

In the embodiment of the present disclosure, a sampling frequency, i.e., a duration of the time window, may be set according to an actual duration of the original physiological signal, to ensure that the original physiological signal may be divided evenly. In addition, compared with signal segmentation in a non-overlapping manner, overlapping time windows may obtain more data. Moreover, since the waveform of the original physiological signal may have a phase difference, capturing the waveform of the original physiological signal may not always start from a P-wave or T-wave position of the original physiological signal, but may start from any position of the original physiological signal. Using overlapping adjacent time windows can simulate the original physiological signal more accurately, thereby improving the accuracy of subsequent processing.

In specific implementation, for an original physiological signal with a time length of T seconds and including k pieces of data, a time window of L seconds may be set, with a delay duration being o seconds, and the number of pieces of data within o seconds being y. The original physiological signal is divided into the plurality of physiological signal segments according to the time window, and each physiological signal segment has a time length of L seconds and includes m pieces of data, where L is smaller than T, o is smaller than L, m is smaller than k, and y is smaller than m.

FIG. 12 is a schematic diagram of a time window provided according to an embodiment of the present disclosure. In FIG. 12, time windows t₀ and t₁ are adjacent, and the delay duration is o seconds.

As an example, each piece of data in the original physiological signal with a time length of T seconds and including k pieces of data is defined as {x₀, x₁, ..., xₖ₋₁}. The original physiological signal is divided into n physiological signal segments with a time window of L, and each physiological signal segment is defined as t₀, t₁, ..., tₙ₋₁. Moreover, t₀ = {x₀, x₁, ..., xₘ₋₁}, t₁ = {x_{y}, x_{y+1}, ..., x_{y+m-1}}, ..., tₙ₋₁ = {xₖ₋ₘ, xₖ₋ₘ₊₁, ..., xₖ₋₁}.

In the embodiments of the present disclosure, time windows of the same duration are used to segment the obtained original physiological signal to obtain a plurality of shorter-duration physiological signal segments with overlapping time windows, facilitating subsequent purification processing.

At a second step, the plurality of physiological signal segments are inputted into a pre-trained neural network model for purification processing to obtain a target physiological signal. The neural network model is trained using a true value of the original physiological signal as a label.

In the embodiments of the present disclosure, the true value is a purified physiological signal obtained by performing band-pass filtering on the original physiological signal, which removes noise from the original physiological signal. In specific implementation, band-pass filtering can be applied to each physiological signal segment based on the measured physiological signal value of the physiological signal segment, with the band-pass frequency band range set to (Hr-5, Hr + 5). The signal obtained after band-pass filtering is used as the true value. Hr is the physiological signal value.

As an example, band-pass filtering on the physiological signal segments can be implemented by the following equation (1): ${Ft}_{i} = FIR \left({t}_{i}\right) , i = 0 , 1 , 2 , … , n - 1$

In equation (1), FIR denotes a signal filter used, which may be signal filtering methods such as ideal band-pass filtering, Butterworth filtering, and IIR filtering. The frequency band range is from Hr-5 to Hr+5. Hr denotes a true physiological signal value of the physiological signal segment tᵢ. Ftᵢ is a signal obtained after band-pass filtering is performed on the physiological signal segment tᵢ, i.e., a true value corresponding to the physiological signal segment tᵢ.

In some embodiments, when the true physiological signal value cannot be determined, a narrow frequency band range cannot be used for filtering. Instead, a common physiological signal value range may be selected as the frequency band range. Moreover, in order to account for the physiological signal value range across different age groups and different situations, a wider frequency band range may be used.

As an example, the original physiological signal is an original electrocardiogram signal. The physiological signal value may be a heart rate value, and the physiological signal value range may be a heart rate range and the heart rate range is a normal human heart rate range, such as [40, 200]. Here, 40 refers to that a heart rate of 40 beats per minute, and 200 refers to a heart rate of 200 beats per minute. Since heart rate ranges of athletes and newborns need to be considered, a wider heart rate range can be selected as the frequency band range.

In some embodiments, the signal after band-pass filtering may be used as the true value (i.e., label) during the training of the neural network model, and the plurality of physiological signal segments may be used as the training data for the neural network model. The neural network model is iteratively trained based on the training data and the label to obtain a trained neural network model. In specific implementation, a plurality of pieces of training data may be inputted into the neural network model to obtain a plurality of prediction signals. A parameter of the neural network model is adjusted using a loss function based on each prediction signal and its corresponding label, until the prediction signal and the true value are substantially identical, to obtain the trained neural network model.

The loss function may be any loss function such as a Mean Squared Error (MSE) function or a cross-entropy loss function.

In the embodiments of the present disclosure, after the neural network model is pre-trained, the plurality of physiological signal segments may be inputted into the pre-trained neural network model for purification processing. The specific implementation may include: performing a down-sampling operation on the plurality of physiological signal segments based on a Focus down-sampling network structure to obtain sampled data groups; concatenating the sampled data groups to obtain first concatenated data; performing a temporal filtering operation on the concatenated data respectively based on a plurality of filtering intervals to obtain a plurality of pieces of filtered data; concatenating the plurality of pieces of filtered data to obtain second concatenated data; and performing encoding and decoding processing on the second concatenated data to obtain the target physiological signal.

In some embodiments, the neural network model may be any model capable of implementing signal purification (filtering). Exemplarily, the neural network model may include at least one of an electroencephalogram (EEGNet) model, a Generative Adversarial Nets (GAN) model, or a Transformer model. Moreover, the neural network model may include a Focus down-sampling unit, a temporal filtering unit, an encoding unit, a decoding unit, and two concatenation units. The Focus down-sampling unit adopts a Focus down-sampling structure and is used for performing the down-sampling operation on the physiological signal segment. The temporal filtering unit is used for performing the temporal filtering operation on the first concatenated data. The encoding unit and the decoding unit are used for performing the encoding and decoding processing on the second concatenated data.

In specific implementation, the plurality of physiological signal segments may be inputted into the Focus down-sampling unit for down-sampling operation respectively to obtain sampled data groups corresponding to each physiological signal segment. Then, via the first concatenation unit, the sampled data groups corresponding to the same physiological signal segment are concatenated to obtain a first concatenated data corresponding to each physiological signal segment. The first concatenated data is inputted into the temporal filtering unit, and temporal filtering processing of different kernel sizes is applied across a plurality of different filtering intervals to obtain a plurality of pieces of filtered data corresponding to each physiological signal segment. Next, via the second concatenation unit, the plurality of pieces of filtered data corresponding to the same physiological signal segment are concatenated to obtain second concatenated data corresponding to each physiological signal segment. The second concatenated data is inputted into the encoding unit for encoding processing, and then the encoding result is inputted into the decoding unit for decoding processing, to obtain a target physiological signal segment corresponding to each physiological signal segment. The plurality of target physiological signal segments are concatenated to obtain the target physiological signal, i.e., the purified signal of the original physiological signal.

As an example, the down-sampling operation of the Focus down-sampling unit involves sampling the physiological signal segment at intervals. Suppose the physiological signal segment is defined as T = [t₀, t₁, ..., tₙ]. If sampling at a time interval of 1, a down-sampling value sequences are T0 = [t₀, t₂, t₄, ...], T1 = [t₁, t₃, t₅, ...]. In this case, the Focus down-sampling unit includes two down-sampling value sequences T0 and T1. If sampling at a time interval of 2, down-sampling value sequences are T0 = [t₀, t₃, t₆, ...], T1 = [t₁, t₄, t₇, ...], T2 = [t₂, t₅, t₈, ...]. In this case, the Focus down-sampling unit includes three down-sampling value sequences T0, T1, and T2. The same logic applies to other time intervals. That is, the Focus down-sampling unit may include at least two down-sampling value sequences. For each physiological signal segment, the at least two down-sampling value sequences are used to obtain at least two sampled data groups corresponding to the physiological signal segment. Then, the at least two sampled data groups corresponding to the same physiological signal segment are concatenated to obtain the first concatenated data corresponding to each physiological signal segment.

As an example, the temporal filtering unit includes a plurality of filter kernels of different sizes, which are used for performing spatial filtering of different sizes on the inputted first concatenated data along the time dimension. This process is analogous to convolution applied to two-dimensional image filtering in the neural network model. Moreover, larger filter kernels are more effective at extracting a low-frequency signal, and smaller filter kernels are more effective at extracting a high-frequency signal. In practice, a size of the filter kernel may be set or the filter kernel to be used may be selected as desired. For example, it is assumed that the temporal filtering unit includes three filter kernels. A filter kernel of k0 with size [1,64], a filter kernel of k1 with size [1,128], and a filter kernel of k2 with size [1,256]. Moreover, for the same physiological signal segment, the number of pieces of filtered data obtained after processing by the temporal filtering unit is the same as the number of filter kernels. The plurality of pieces of filtered data corresponding to the physiological signal segment are concatenated by the second concatenation unit using a set of spatial filters. Specifically, this is accomplished by assigning different weights to the plurality of pieces of filtered data, and combining them to obtain the second concatenated data corresponding to the physiological signal segment.

As an example, the encoding unit may include a convolution layer, a Batch Normalization (BN) layer, a pooling layer, and an activation layer. The decoding unit may include a deconvolution layer, a BN layer, an unpooling layer, and an activation layer. In specific implementation, the second concatenated data is inputted into the encoding unit: first, feature extraction is performed through the convolution layer to obtain a first result; the first result is inputted into the BN layer to normalize the distribution of data in the first result to obtain a second result; the second result is inputted into the pooling layer for down-sampling processing to obtain a third result; the third result is then inputted into the activation layer to obtain an encoding result. The encoding result is inputted into the decoding unit: first, up-sampling processing is performed through the deconvolution layer to obtain a fourth result; the fourth result is inputted into the BN layer to normalize the distribution of data in the fourth result to obtain a fifth result; the fifth result is inputted into the unpooling layer for up-sampling processing to obtain a sixth result; the sixth result is then inputted into the activation layer to obtain the target physiological signal segment.

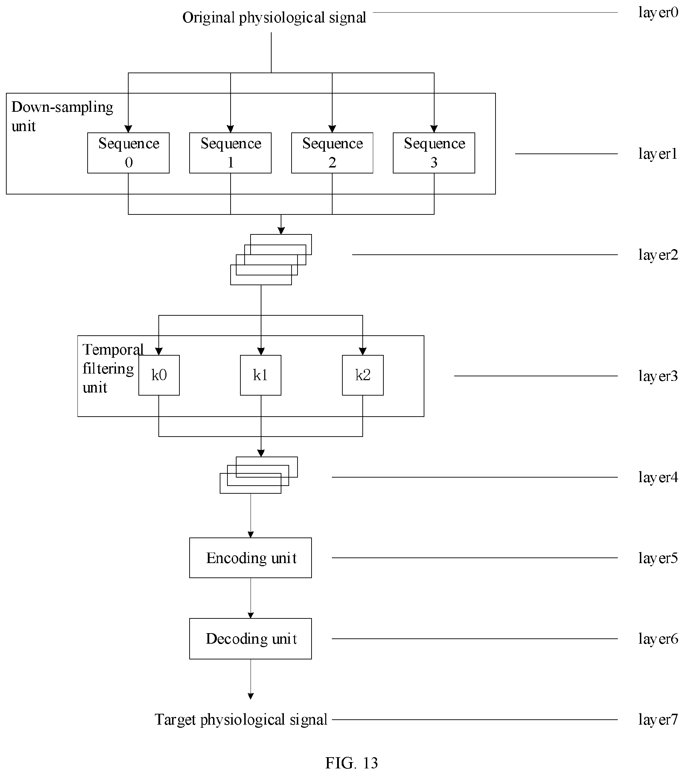

Exemplarily, FIG. 13 is a schematic diagram of a structure of a neural network model provided according to an embodiment of the present disclosure. The neural network model includes 6 layers. Layer0 is the input of the neural network model, i.e., the original physiological signal (divided into the plurality of physiological signal segments). Layer1 is a Focus down-sampling unit, which includes four down-sampling value sequences: Focus0, Focus1, Focus2, and Focus3 that are denoted as Sequence 0 to Sequence 3 in FIG. 13. It can perform the down-sampling processing on a signal without losing data information. Layer2 is a first concatenation unit, configured to concatenate the output of layer1. Layer3 is a temporal filtering unit, which includes three filter kernels k0, k1, and k2, and is configured to perform temporal filtering processing of different sizes on the output of layer2. Layer4 is the second concatenation unit, which uses a set of spatial filters to concatenate the output of layer3. Layer5 is the encoding unit, which includes a convolution layer, a BN layer, a pooling layer, and an activation layer. Layer6 is the decoding unit, which includes a deconvolution layer, a BN layer, an unpooling layer, and an activation layer. Layer7 is the output of the neural network model, i.e., the target physiological signal. Layer0 to layer7 are indicated in FIG. 13.

Taking the original physiological signal as an electrocardiogram signal as an example, an electrocardiogram signal processing method provided in the embodiments of the present disclosure may include: obtaining an electrocardiogram signal with a time length of T, segmenting the electrocardiogram signal into n electrocardiogram slice signals ti (i=0,1,...,n-1) with overlapping time windows, and performing purification processing on the n electrocardiogram slice signals using the neural network model to obtain a target electrocardiogram signal.

In the embodiments of the present disclosure, the original physiological signal is sliced, and the obtained physiological signal segments are inputted into the neural network model for purification processing, i.e., filtering out noise from the original physiological signal to obtain a target physiological signal. The target physiological signal exhibits a higher signal-to-noise ratio, enabling the extraction of higher-quality features, improving the accuracy of feature extraction, and providing a more accurate judgment basis for medical diagnosis. Moreover, the slicing processing is performed on the original physiological signal before the purification, and the purification processing is performed on the physiological signal segments, which can improve purification accuracy and achieve a better denoising effect.

It can be understood that performing the purification processing on the original physiological signal in the embodiments of the present disclosure may also be called denoising processing, and both the purification processing and the denoising processing are essentially the same in technology. For example, the denoising processing is performed on a electrocardiogram signal of a user in the first embodiment, i.e., reference can be made to the processing method in the following steps.

A HRV feature extraction method provided in embodiments of the present disclosure may include the following steps.

At a first step, an original electrocardiogram signal is obtained, and segmentation processing is performed on the original electrocardiogram signal to obtain a plurality of electrocardiogram slice signals that overlap.

At a second step, the plurality of electrocardiogram slice signals are inputted into a pre-trained neural network model for purification processing to obtain a target electrocardiogram signal. The neural network model is trained using the purified electrocardiogram signal of the original electrocardiogram signal as a label, and the purified electrocardiogram signal is obtained by performing the band-pass filtering on the original electrocardiogram signal.

It should be noted that the implementation processes of the first step and the second step are similar to those in the previous embodiments, except that the original physiological signal is replaced with the original electrocardiogram signal. Therefore, the specific implementation processes of the first step and the second step can refer to the relevant description of the previous embodiments, and details are omitted in this embodiment.

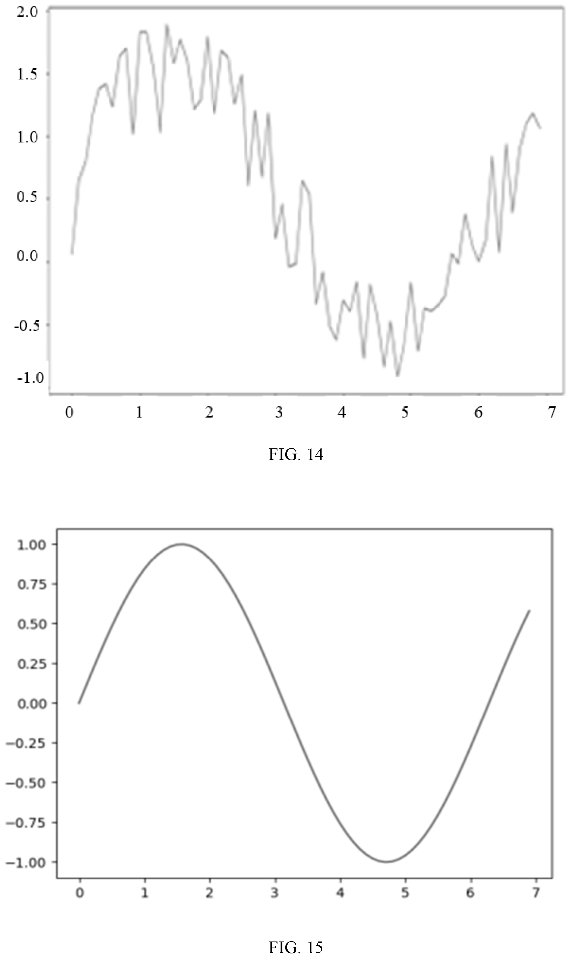

Exemplarily, referring to FIG. 14 and FIG. 15, FIG. 14 is a schematic diagram of an original electrocardiogram signal provided according to an embodiment of the present disclosure, and FIG. 15 is a schematic diagram of a target electrocardiogram signal provided according to an embodiment of the present disclosure. The electrocardiogram slice signal inputted to the neural network model is an ECG signal with glitches as shown in FIG. 14, and the target electrocardiogram signal outputted by the neural network model is a smoother ECG signal as shown in FIG. 15. During the training of the neural network model, a real desired waveform is required, but the collected ECG signal often resemble the form shown in FIG. 14. Therefore, while ECG data is collected, a real-time heart rate value may be recorded by a pulse oximeter device or an ECG data collection device with heart rate measurement. Based on the real-time heart rate value, band-pass filtering with a relatively narrow band can be applied to the ECG signal in FIG. 14 to obtain the ECG signal in FIG. 15. During the model training process, the ECG signal in FIG. 14 may be used as the input of the neural network model, and the ECG signal in FIG. 15 may be used as a label (i.e., a true value) to train the neural network model, so that the trained neural network model may purify an ECG signal similar to the ECG signal in FIG. 14 to obtain an ECG signal similar to the ECG signal in FIG. 15.

At a third step, a peak position and a valley position of the target electrocardiogram signal are determined, and a HRV feature is determined based on the peak position and the valley position.

Heart Rate Variability (HRV) refers to a time difference between heartbeats, and is an important indicator reflecting regulation of an autonomic nervous system, as well as an important indicator for evaluating cardiac health.

In specific implementation, according to the peak position and the valley position of the target electrocardiogram signal, a peak-to-peak interval between every two adjacent peaks may be determined, or a valley-to-valley interval between every two adjacent valleys may be determined. An HRV signal may be formed based on the peak-to-peak interval or the valley-to-valley interval, and various HRV features such as a time-domain feature, a frequency-domain feature, and a Poincaré feature may be extracted from the HRV signal to assist in medical diagnosis.

Exemplarily, referring to FIG. 16, FIG. 16 is a schematic diagram of another target electrocardiogram signal provided according to an embodiment of the present disclosure. First, a peak position and a valley position in the ECG signal are identified, and a time length (in milliseconds) between every two adjacent peaks or between every two adjacent valleys is calculated. A time signal formed by the time length between every two adjacent peaks or between every two adjacent valleys is the HRV signal. Various HRV features may be extracted from the HRV signal, such as more than time-domain features (dozens of types), frequency-domain features (commonly about 7 to 8 types), and Poincaré features.

The HRV feature extraction method provided in the embodiments of the present disclosure includes: obtaining the original electrocardiogram signal, and performing the segmentation processing on the original electrocardiogram signal to obtain the plurality of electrocardiogram slice signals that overlap; inputting the plurality of electrocardiogram slice signals into the pre-trained neural network model for purification processing to obtain the target electrocardiogram signal, where the neural network model is trained using the purified electrocardiogram signal of the original electrocardiogram signal as a label, and the purified electrocardiogram signal is obtained by performing the band-pass filtering on the original electrocardiogram signal; and determining the peak position and the valley position of the target electrocardiogram signal, and determining the HRV feature based on the peak position and the valley position. The above method performs the purification processing on the original electrocardiogram signal using the pre-trained neural network model to filter out the noise from the original electrocardiogram signal, and obtains a purer target electrocardiogram signal. Then, the HRV feature is extracted based on the target electrocardiogram signal, which can reduce the interference of noise, improve the accuracy of feature extraction, and thereby improve the accuracy of medical diagnosis. Moreover, the slicing processing is performed on the original electrocardiogram signal before the purification, and the slicing processing is performed on the electrocardiogram slice signal, which can improve the purification accuracy and achieve a better denoising effect.

It should be noted that the above embodiments are exemplified by using an electrocardiogram signal as the original physiological signal, to explain the HRV feature extraction. When the original physiological signal is an electroencephalogram signal, the heart rate value may be replaced with the frequency band of the electroencephalogram signal, such as a Delta waveband (with band-pass smaller than 4 Hz), a Theta oscillation waveband (with band-pass of 4 Hz to 7 Hz), a α waveband (with band-pass of 7 Hz to12 Hz), a Beta waveband (with band-pass of 12 Hz to 30 Hz), and a Gamma waveband (with band-pass of 30 Hz to50 Hz or higher). Moreover, waveforms in different frequency bands have different effects on in human functions. Therefore, the above processing can also be applied to the electroencephalogram signal to train a neural network model capable of purifying the electroencephalogram signal. The neural network model can then be used to purify the original electroencephalogram signal to obtain the target electroencephalogram signal, from which features related to medical diagnosis can be extracted to provide a basis for medical diagnosis. For a specific implementation when the original physiological signal is an electroencephalogram signal, reference may be made to the relevant description in the above embodiments, and details are omitted in this embodiment.

An embodiment of the present disclosure provides a human-factor intelligence-based physiological signal processing apparatus. The apparatus may include: a first obtaining module configured to obtain an original physiological signal; a first segmentation module configured to perform slicing processing on the original physiological signal to obtain a plurality of physiological signal segments; and a first processing module configured to input the plurality of physiological signal segments into a pre-trained neural network model for purification processing to obtain a target physiological signal. The neural network model is trained using a true value of the original physiological signal as a label.

According to an embodiment of the present disclosure, the first segmentation module is further configured to: segment the original physiological signal using a time window. At least adjacent time windows overlap.

According to an embodiment of the present disclosure, each time window remains a consistent duration.

According to an embodiment of the present disclosure, the true value is obtained by performing band-pass filtering on the original physiological signal.

According to an embodiment of the present disclosure, the first processing module is further configured to: perform a down-sampling operation on the plurality of physiological signal segments based on a Focus down-sampling network structure to obtain sampled data groups; concatenate the sampled data groups to obtain first concatenated data; perform temporal filtering operations on the concatenated data respectively based on a plurality of filtering intervals to obtain a plurality of pieces of filtered data; concatenate the plurality of pieces of filtered data to obtain second concatenated data; and perform encoding and decoding processing on the second concatenated data to obtain the target physiological signal.

According to an embodiment of the present disclosure, the original physiological signal includes an electrocardiogram signal and an electroencephalogram signal.

According to an embodiment of the present disclosure, the neural network model includes at least one of an EEGNet model, a GAN model, or a Transformer model.

By applying the human-factor intelligence-based physiological signal processing method provided in the embodiments of the present disclosure, the purification processing is performed on the original physiological signal through a pre-trained neural network model to filter out the noise from the original physiological signal, thereby obtaining a purer target physiological signal. Then, feature extraction is performed based on the target physiological signal, which can reduce the interference of noise, improve the accuracy of feature extraction, and thereby improve the accuracy of medical diagnosis. Moreover, the slicing processing is performed on the original physiological signal before the purification, and the purification processing is performed on the physiological signal segment, which can improve the purification accuracy and achieve a better denoising effect.

The above presents a schematic solution of the human-factor intelligence-based physiological signal processing apparatus according to the embodiments of the present disclosure. It should be noted that the technical solution of the human-factor intelligence-based physiological signal processing apparatus shares the same concept as the technical solution of the human-factor intelligence-based physiological signal processing method as described above. For detailed content not described in the technical solutions of the human-factor intelligence-based physiological signal processing apparatus, reference can be made to the description of the technical solutions of the human-factor intelligence-based physiological signal processing method as described above.

An embodiment of the present disclosure provides an HRV feature extraction apparatus. The apparatus may include: a second obtaining module configured to obtain an original electrocardiogram signal; a second segmentation module configured to perform segmentation processing on the original electrocardiogram signal to obtain a plurality of electrocardiogram slice signals that overlap; a second processing module configured to input the plurality of electrocardiogram slice signals into a pre-trained neural network model for purification processing to obtain a target electrocardiogram signal, where the neural network model is trained using the purified electrocardiogram signal of the original electrocardiogram signal as a label, and the purified electrocardiogram signal is obtained by performing band-pass filtering on the original electrocardiogram signal; and a determination module configured to determine a peak position and a valley position of the target electrocardiogram signal, and determine an HRV feature based on the peak position and the valley position.

According to an embodiment of the present disclosure, the second segmentation module is further configured to: segment the original electrocardiogram signal using a time window. At least adjacent time windows overlap.

According to an embodiment of the present disclosure, the second processing module is further configured to: perform a down-sampling operation on the plurality of electrocardiogram slice signals based on a Focus down-sampling network structure to obtain sampled data groups; concatenate the sampled data groups to obtain first concatenated data; perform temporal filtering operations on the concatenated data respectively based on a plurality of filtering intervals to obtain a plurality of pieces of filtered data; concatenate the plurality of pieces of filtered data to obtain second concatenated data; and perform encoding and decoding processing on the second concatenated data to obtain the target electrocardiogram signal.

By applying the HRV feature extraction method provided in the embodiments of the present disclosure, the purification processing is performed on the original electrocardiogram signal using the pre-trained neural network model to filter out the noise from the original electrocardiogram signal, thereby obtaining a purer target electrocardiogram signal. Then, an HRV feature is extracted based on the target electrocardiogram signal, which can reduce the interference of noise, improve the accuracy of feature extraction, and thereby improve the accuracy of medical diagnosis. Moreover, the slicing processing is performed on the original electrocardiogram signal before the purification, and the purification processing is performed on the electrocardiogram slice signal, which can improve the purification accuracy and achieve a better denoising effect.

The above presents a schematic solution of the HRV feature extraction apparatus according to the embodiments of the present disclosure. It should be noted that the technical solution of the HRV feature extraction apparatus shares the same concept as the HRV feature extraction method as described above. For detailed content not described in the technical solutions of the HRV feature extraction apparatus, reference can be made to the description of the technical solutions of the HRV feature extraction method as described above.

FIG. 17 is a schematic diagram of a structure of an electronic device provided according to an embodiment of the present disclosure. The electronic device 3100 includes: a memory 3101, a processor 3102, and a computer program stored in the memory 3101 and executable on the processor 3102. The processor 3102, when executing the computer program, implements the training method for the classification model for multimodal human physiological data and the classification method for multimodal human physiological data as shown in the first embodiment, the method for generating electroencephalogram signal sample data and the training method for the physiological state prediction model as shown in the second embodiment, the human-factor intelligence-based physiological signal processing method as shown in the third embodiment, and the human-factor intelligence-based physiological signal processing method or the HRV feature extraction method as shown in the fourth embodiment.

The present disclosure further provides a computer-readable storage medium, storing a computer program. The program, when executed by a processor, implements the training method for the classification model for multimodal human physiological data and the classification method for multimodal human physiological data as shown in the first embodiment, the method for generating electroencephalogram signal sample data and the training method for the physiological state prediction model as shown in the second embodiment, the human-factor intelligence-based physiological signal processing method as shown in the third embodiment, and the human-factor intelligence-based physiological signal processing method or the HRV feature extraction method as shown in the fourth embodiment.

An implementation of the present disclosure provides a computer program product, including an instruction. The instruction, when executed by a processor of a computer device, causes the computer device to execute the training method for the classification model for multimodal human physiological data and the classification method for multimodal human physiological data as shown in the first embodiment, the method for generating electroencephalogram signal sample data and the training method for the physiological state prediction model as shown in the second embodiment, the human-factor intelligence-based physiological signal processing method as shown in the third embodiment, and the human-factor intelligence-based physiological signal processing method or the HRV feature extraction method as shown in the fourth embodiment.

Those skilled in the art should be able to realize that the method steps of each example described in conjunction with the embodiments disclosed herein can be implemented by electronic hardware, computer software, or a combination of the two. In order to clearly illustrate the interchangeability of electronic hardware and software, the composition and steps of each example have been generally described in terms of functions in the above description. Whether these functions are executed by electronic hardware or software depends on the specific application and design constraint conditions of the technical solutions. Those skilled in the art can use different methods to implement the described functions for each specific application, but such implementation should not be considered as going beyond the scope of the present disclosure.

So far, the technical solutions of the present disclosure have been described in conjunction with the preferred implementations shown in the accompanying drawings. However, those skilled in the art can easily understand that the scope of the present disclosure is obviously not limited to these specific implementations. Without departing from the principle of the present disclosure, those skilled in the art can make equivalent changes or substitutions to relevant technical features, and the technical solutions after these changes or substitutions will all fall within the scope of the present disclosure.

## Claims

**1.** A classification model for multimodal human physiological data, the classification model comprising: a multi-headed self-attention module, a normalization module, a fusion expert system, and a decision module, wherein:
the fusion expert system comprises an electroencephalogram expert subsystem, an electrocardiogram expert subsystem, an electrodermal activity expert subsystem, and a multimodal synchronous fusion expert subsystem;
the multi-headed self-attention module is configured to perform feature extraction on inputted multimodal synchronous data and output the extracted feature data to the normalization module, the multimodal synchronous data comprising: a physiological indicator of a user, and the physiological indicator comprising electroencephalogram data, electrocardiogram data, and electrodermal activity data;
the normalization module is configured to generate normalized feature data based on the extracted feature data and output the normalized feature data to the fusion expert system;
the electroencephalogram expert subsystem is configured to perform a classification task based on the normalized feature data corresponding to the electroencephalogram data of the user to obtain a first classification result;
the electrocardiogram expert subsystem is configured to perform a classification task based on the normalized feature data corresponding to the electrocardiogram data of the user to obtain a second classification result;
the electrodermal activity expert subsystem is configured to perform a classification task based on the normalized feature data corresponding to the electrodermal activity data of the user to obtain a third classification result;
the multimodal synchronous fusion expert subsystem is configured to perform a classification task based on the normalized feature data corresponding to the multimodal synchronous data to obtain a fourth classification result; and
the decision module is configured to calculate a final classification result based on the first classification result, the second classification result, the third classification result, the fourth classification result, and a weight corresponding to each of the first classification result, the second classification result, the third classification result, and the fourth classification result.

**2.** The classification model for multimodal human physiological data according to claim 1, wherein the step of calculating the final classification result based on the first classification result, the second classification result, the third classification result, the fourth classification result, and the weight corresponding to each of the first classification result, the second classification result, the third classification result, and the fourth classification result comprises:
calculating the final classification result according to the following equation: $G = {W}_{1} * {f}_{1} + {W}_{2} * {f}_{2} + {W}_{3} * {f}_{3} + {W}_{4} * {f}_{4} ,$
wherein G denotes the final classification result; f₁, f₂, f₃ and f₄ respectively denote the first classification result, the second classification result, the third classification result, and the fourth classification result; and W₁, W₂, W₃, and W₄ each denote a weight.

**3.** A training method for a classification model for multimodal human physiological data, applicable to the classification model for multimodal human physiological data according to any one of claims 1 to 2, wherein the training method comprises:
training the electroencephalogram expert subsystem and the multi-headed self-attention module using an electroencephalogram training set, and freezing a weight of the electrocardiogram expert subsystem, a weight of the electrodermal activity expert subsystem, and a weight of the multimodal synchronous fusion expert subsystem;
training the electrocardiogram expert subsystem using an electrocardiogram training set, and freezing a weight of the multi-headed self-attention module, a weight of the electroencephalogram expert subsystem, the weight of the electrodermal activity expert subsystem, and the weight of the multimodal synchronous fusion expert subsystem;
training the electrodermal activity expert subsystem using an electrodermal activity training set, and freezing the weight of the multi-headed self-attention module, the weight of the electroencephalogram expert subsystem, the weight of the electrocardiogram expert subsystem, and the weight of the multimodal synchronous fusion expert subsystem; and
training the classification model using a multimodal random masking training set, to adjust the weight of the multi-headed self-attention module, the weight of the electroencephalogram expert subsystem, the weight of the electrocardiogram expert subsystem, the weight of the electrodermal activity expert subsystem, and the weight of the multimodal synchronous fusion expert subsystem.

**4.** The training method for a classification model for multimodal human physiological data according to claim 3, wherein:
the electroencephalogram training set comprises original electroencephalogram signal sample data and target electroencephalogram signal sample data; and
said training the electroencephalogram expert subsystem using the electroencephalogram training set comprises:
obtaining the target electroencephalogram signal sample data, wherein the target electroencephalogram signal sample data is determined based on an electroencephalogram signal prior feature, and wherein the electroencephalogram signal prior feature is obtained based on the original electroencephalogram signal sample data;
performing auxiliary training on a to-be-trained electroencephalogram expert subsystem based on the target electroencephalogram signal sample data to obtain an auxiliary electroencephalogram expert subsystem; and
training the auxiliary electroencephalogram expert subsystem based on the original electroencephalogram signal sample data to obtain a trained electroencephalogram expert subsystem.

**4.** The training method for a classification model for multimodal human physiological data according to claim 3, wherein said obtaining the target electroencephalogram signal sample data comprises:
processing the original electroencephalogram signal sample data to obtain the electroencephalogram signal prior feature; and
constructing first target electroencephalogram signal sample data according to the electroencephalogram signal prior feature; or
processing the original electroencephalogram signal sample data to obtain the electroencephalogram signal prior feature; and
merging the original electroencephalogram signal sample data with the electroencephalogram signal prior feature to obtain second target electroencephalogram signal sample data.

**7.** The training method for a classification model for multimodal human physiological data according to claim 5 or 6, wherein said processing the original electroencephalogram signal sample data to obtain the electroencephalogram signal prior feature comprises:
processing the original electroencephalogram signal sample data according to a predetermined feature indicator to obtain a feature value corresponding to the predetermined feature indicator as the electroencephalogram signal prior feature.

**8.** The training method for a classification model for multimodal human physiological data according to claim 5, wherein said performing the auxiliary training on the to-be-trained electroencephalogram expert subsystem based on the target electroencephalogram signal sample data to obtain the auxiliary electroencephalogram expert subsystem comprises:
inputting the first target electroencephalogram signal sample data into the to-be-trained electroencephalogram expert subsystem for prediction to obtain a first fine-grained classification prediction result representing a physiological state; and
adjusting a model parameter of the to-be-trained electroencephalogram expert subsystem based on the first fine-grained classification prediction result to obtain the auxiliary electroencephalogram expert subsystem.

**9.** The training method for a classification model for multimodal human physiological data according to claim 6, wherein said performing the auxiliary training on the to-be-trained electroencephalogram expert subsystem based on the target electroencephalogram signal sample data to obtain the auxiliary electroencephalogram expert subsystem comprises:
inputting the second target electroencephalogram signal sample data into the to-be-trained electroencephalogram expert subsystem for prediction to obtain a second fine-grained classification prediction result and a first coarse-grained classification prediction result that represent a physiological state; and
adjusting a model parameter of the to-be-trained electroencephalogram expert subsystem based on the second fine-grained classification prediction result and the first coarse-grained classification prediction result to obtain the auxiliary electroencephalogram expert subsystem.

**10.** The training method for a classification model for multimodal human physiological data according to claim 8 or 9, wherein said training the auxiliary electroencephalogram expert subsystem based on the original electroencephalogram signal sample data to obtain the trained electroencephalogram expert subsystem comprises:
inputting the original electroencephalogram signal sample data into the auxiliary electroencephalogram expert subsystem for prediction to obtain a second coarse-grained classification prediction result representing the physiological state; and
adjusting a model parameter of the auxiliary electroencephalogram expert subsystem based on the second coarse-grained classification prediction result to obtain the trained electroencephalogram expert subsystem.

**11.** The training method for a classification model for multimodal human physiological data according to claim 3, the method further comprising:
generating electroencephalogram data containing time-domain information, frequency-domain information, and spatial information according to an electroencephalogram signal of a user and positions of electrodes corresponding to the electroencephalogram signal to obtain the electroencephalogram training set;
performing denoising processing on an electrocardiogram signal of the user and aligning the denoised electrocardiogram signal with the electroencephalogram signal of the user according to temporal information to generate the electrocardiogram training set;
performing denoising processing on an electrodermal activity signal of the user and aligning the denoised electrodermal activity signal with the electroencephalogram signal of the user according to the temporal information to generate the electrodermal activity training set; and
performing random masking processing on the electroencephalogram training set, the electrocardiogram training set, and the electrodermal activity training set, to generate the multimodal random masking training set.

**12.** The training method for a classification model for multimodal human physiological data according to claim 11, wherein the step of generating the electroencephalogram data containing the time-domain information, the frequency-domain information, and the spatial information according to the electroencephalogram signal of the user and the positions of the electrodes corresponding to the electroencephalogram signal to obtain the electroencephalogram training set comprises:
performing fast Fourier transform on the electroencephalogram signal of the user to extract the frequency-domain information;
selecting data within a frequency band of interest from the frequency-domain information;
obtaining a multispectral-like image based on different leads according to the data within the frequency band of interest and the positions of the electrodes at which the electroencephalogram signal is collected; and
segmenting the multispectral-like image and generating the electroencephalogram data of the user according to segmented data.

**13.** The training method for a classification model for multimodal human physiological data according to claim 12, wherein the step of obtaining the multispectral-like image based on the different leads according to the data within the frequency band of interest and the positions of the electrodes at which the electroencephalogram signal is collected comprises:
projecting the positions of the electrodes at which the electroencephalogram signal is collected from a three-dimensional space to a two-dimensional surface to obtain two-dimensional position information of each electrode;
calculating distances between each electrode and other surrounding electrodes according to the two-dimensional position information; and
for data of each lead in the data within the frequency band of interest, setting corresponding weights for data of other leads according to the distances, to obtain the multispectral-like image based on the different leads.

**14.** The training method for a classification model for multimodal human physiological data according to claim 12, wherein the step of segmenting the multispectral-like image and generating electroencephalogram data of the user according to the segmented data comprises:
segmenting the multispectral-like image to obtain *N = H * W* / *P²* patches of a same size, wherein H, W, C, and P denote a height, width, number of channels of the image, and a size of each of the *N = H * W* / *P²* patches, respectively;
for each patch, flattening the patch into a vector, obtaining Patch embedding through linear projection, and adding an [I_CLS] token as position encoding of the patch, to obtain the segmented data corresponding to the patch; and
generating the electroencephalogram data of the user according to the segmented data.

**15.** The training method for a classification model for multimodal human physiological data according to claim 13, wherein the step of projecting the positions of the electrodes at which the electroencephalogram signal is collected from the three-dimensional space to the two-dimensional surface to obtain the two-dimensional position information of the electrode comprises:
performing azimuthal equidistant projection in the Cartesian coordinate system to transform the positions of the electrodes at which the electroencephalogram signal is collected from the three-dimensional space to the two-dimensional surface, wherein the transformed data of respective leads retain a spatial topological relationship.

**16.** The training method for a classification model for multimodal human physiological data according to claim 11, wherein said performing the denoising processing on the electrocardiogram signal of the user comprises:
obtaining an original electrocardiogram signal and performing slicing processing on the original electrocardiogram signal to obtain a plurality of electrocardiogram signal segments; and
inputting the plurality of electrocardiogram signal segments into a pre-trained neural network model for denoising processing to obtain a target electrocardiogram signal, wherein the neural network model is trained using a true value of the original electrocardiogram signal as a label.

**17.** The training method for a classification model for multimodal human physiological data according to claim 16, wherein said performing the slicing processing on the original electrocardiogram signal comprises:
segmenting the original electrocardiogram signal using a time window, wherein at least adjacent time windows overlap.

**18.** The training method for a classification model for multimodal human physiological data according to claim 17, wherein said inputting the plurality of electrocardiogram signal segments into the pre-trained neural network model for denoising processing comprises:
performing a down-sampling operation on the plurality of electrocardiogram signal segments based on a Focus down-sampling network structure to obtain sampled data groups;
concatenating the sampled data groups to obtain first concatenated data;
performing temporal filtering operations on the concatenated data respectively based on a plurality of filtering intervals to obtain a plurality of pieces of filtered data;
concatenating the plurality of pieces of filtered data to obtain second concatenated data; and
performing encoding processing and decoding processing on the second concatenated data to obtain the target electrocardiogram signal.

**19.** A classification method for multimodal human physiological data, wherein the classification method comprises performing a classification task using the classification model for multimodal human physiological data according to any one of claims 1 to 2.

**20.** The classification method according to claim 19, wherein prior to performing the classification task using the classification model for multimodal human physiological data, the method comprises:
performing weighting processing on inputted physiological data through a channel attention network to obtain first weighted physiological data, wherein the number of channels of the physiological data is not compressed during the processing of the physiological data by the channel attention network;
performing weighting processing on the first weighted physiological data through a spatial attention network to obtain second weighted physiological data; and
obtaining a type of the physiological data based on the second weighted physiological data.

**21.** The classification method according to claim 20, wherein:
the channel attention network comprises a first sub-network and a second sub-network; and
said performing the weighting processing on the inputted physiological data through the channel attention network to obtain the first weighted physiological data comprises:
inputting the physiological data into the first sub-network to obtain a first channel weight vector, wherein the number of channels of the physiological data is not compressed during the processing of the physiological data by the first sub-network;
inputting the physiological data into the second sub-network to obtain a second channel weight vector, wherein the number of channels of the physiological data is not compressed during the processing of the physiological data by the second sub-network; and
obtaining the first weighted physiological data based on the first channel weight vector, the second channel weight vector, and the physiological data.

**22.** The classification method according to claim 21, wherein said obtaining the first weighted physiological data based on the first channel weight vector, the second channel weight vector, and the physiological data comprises:
performing weighted summation on the first channel weight vector and the second channel weight vector to obtain a target channel weight vector; and
performing the weighting processing on the physiological data using the target channel weight vector to obtain the first weighted physiological data.

**23.** The classification method according to any one of claims 20 to 22, wherein:
the spatial attention network comprises a pooling network and a convolution block; and
said performing the weighting processing on the first weighted physiological data through the spatial attention network to obtain the second weighted physiological data comprises:
compressing the first weighted physiological data along a channel dimension based on the pooling network to obtain third compressed physiological data;
performing convolution processing on the third compressed physiological data through the convolution block to obtain a target spatial weight vector; and
performing the weighting processing on the first weighted physiological data using the target spatial weight vector to obtain the second weighted physiological data.

**24.** The classification method according to any one of claims 20 to 22, wherein said obtaining the type of the physiological data based on the second weighted physiological data comprises:
processing the second weighted physiological data through a physiological data recognition network to obtain the type of the physiological data.

**25.** A computer-readable storage device, storing a computer program, wherein the computer program is capable of being loaded and executed by a processor to implement the method according to any one of claims 3 to 24.
